# EUROPEAN PATENT APPLICATION

(11) **EP 4 825 876 A1**
(43) Date of publication of application: **21.10.2026**
(21) Application number: 25171127.1
(22) Date of filing: 16.04.2025
(51) Int. Cl.: C07K 16/28, A61P 35/00, A61K 39/00

(54) **CFC1 MODULATOR**

(71) Applicant: TRON gGmbH, 55131 Mainz (DE); BioNTech SE, 55131 Mainz (DE)
(72) Inventor: The designation of the inventor has not yet been filed
(74) Representative: D Young & Co LLP

(57) **Abstract**

The present invention relates to a modulator of CFC1 for use in the treatment and/or prevention of neuroendocrine neoplasms including both well-differentiated neuroendocrine tumors (NETs) and poorly differentiated neuroendocrine carcinomas (NECs). In particular, the modulator of CFC1 may be an antibody.

## Description

### FIELD OF THE INVENTION

The present invention relates to a modulator of CFC1 for use in the treatment and/or prevention of neuroendocrine neoplasms including both well-differentiated neuroendocrine tumors (NETs) and poorly differentiated neuroendocrine carcinomas (NECs). In particular, the modulator of CFC1 may be an antibody.

### BACKGROUND TO THE INVENTION

### CFC1

CFC1 is a member of the EGF-CFC protein family and can be cell-associated/membrane bound or can be extracellular and present in soluble form.

CFC1 is known to be a co-receptor that binds Nodal with ALK4/7 receptors, which can result in phosphorylation of SMAD 2/3 that in turn associates with SMAD4 and translocates to the nucleus.

It is also known that CFC1 is involved in regulation of gastrulation, mesoderm induction, and axis formation during embryonic development. Moreover, CFC1 functions as a Nodal co-receptor to establish left right asymmetry of developing organs.

CFC1 is associated with heterotaxy syndrome in humans. Mice with knock-out of CFC1 survive until birth, but often die within the first week of life due to complex cardiac malformations, and typically display severe left-right laterality defects, but do not exhibit the phenotypes associated with pre-gastrulation patterning and differentiation.

However, it is not known to what extent CFC1 is associated with cancer, nor is expression of CFC1 on tumor tissue well characterised. There is a need for therapies that target and treat CFC1-expressing tumours and cancers.

### Neuroendocrine neoplasms

The current treatment for neuroendocrine neoplasms are limited and often depend on the type of tumor. In general, neuroendocrine tumor treatment options might include standard tumor therapies such as surgery, chemotherapy, radiotherapy such as peptide receptor radionuclide therapy (PRRT) and medications such as somatostatin analogues to control excess hormone. However, these treatments are not effective in progressed tumors and metastases. Systemic therapies are primarily used for initial symptom control, particularly for hormone-secreting tumors, but are not curative. Chemotherapy efficacy varies by tumor grade, and recurrence is common. Molecular targeted therapies often result in resistance, while PRRT frequently results in disease progression, sometimes shortly after treatment. Immunotherapy, including checkpoint inhibitors and antibodies against SSTR2 and DLL3, has shown limited efficacy.

Therefore, further treatment options to combat neuroendocrine neoplasm are urgently needed.

### SUMMARY OF THE INVENTION

The present inventors have surprisingly found that CFC1 is expressed on neuroendocrine neoplasms, such as neuroendocrine tumours and neuroendocrine cancer. CFC1 is a member of the EGF-CFC family and acts as a co-receptor for the TGF-beta pathway which is pivotal in controlling cell growth, differentiation, and migration.

The present invention provides a modulator of CFC1 for use in treating neuroendocrine neoplasms.

In some embodiments, the modulator antagonizes CFC1 activity.

In some embodiments, the modulator inhibits CFC1 signalling.

In some embodiments, the modulator inhibits the function of CFC1.

In some embodiments, the modulator is capable of recruiting immune cells.

In some embodiments, the modulator is capable of targeting immune cells to a tumour or cancer, wherein the tumour or cancer expresses CFC1.

In some embodiments, the modulator elicits an immune response. In some embodiments, the modulator elicits an anti-tumoral immune response.

In some embodiments, the modulator is an inhibitor of CFC1.

In some embodiments, the modulator is a binding molecule comprises a CFC1 binding domain.

In some embodiments, the modulator is an inhibitor which is a binding molecule comprising a CFC1 binding domain.

In some embodiments, the binding molecule comprises or consists of an anti-CFC1 antibody or a CFC1-binding antibody fragment thereof.

In some embodiments, the binding molecule comprises an anti-CFC1 antibody. In some embodiments, the binding molecule comprises a CFC1-binding antibody fragment thereof.

In some embodiments, the binding molecule consists of an anti-CFC1 antibody. In some embodiments, the binding molecule consists of a CFC1-binding antibody fragment thereof.

In some embodiments, the binding molecule binds to SEQ ID NO: 1 or a variant having at least 80% sequence identity thereto.

In some embodiments, the binding molecule binds to SEQ ID NO: 2 or a variant having at least 80% sequence identity thereto.

In some embodiments, the binding molecule binds to SEQ ID NO: 3 or a variant having at least 80% sequence identity thereto.

In some embodiments, the binding molecule binds to SEQ ID NO: 4 or a variant having at least 80% sequence identity thereto.

In some embodiments, the antibody or fragment thereof binds to SEQ ID NO: 1 or a variant having at least 80% sequence identity thereto.

In some embodiments, the antibody or fragment thereof binds to SEQ ID NO: 2 or a variant having at least 80% sequence identity thereto.

In some embodiments, the antibody or fragment thereof binds to SEQ ID NO: 3 or a variant having at least 80% sequence identity thereto.

In some embodiments, the antibody or fragment thereof binds to SEQ ID NO: 4 or a variant having at least 80% sequence identity thereto.

In some embodiments, the antibody or fragment thereof comprises a heavy chain variable (VH) domain. In some embodiments, antibody or fragment thereof comprises a light chain variable (VL) domain.

In some embodiments, the antibody or fragment thereof comprises a heavy chain variable (VH) domain and a light chain variable (VL) domain.

In some embodiments, the VH domain comprises heavy chain complementarity determining regions (HCDRs) 1-3, wherein (according to Kabat numbering):
HCDR1 comprises the amino acid sequence according to SEQ ID NO: 5 (SSIYYMC),
HCDR2 comprises the amino acid sequence according to SEQ ID NO: 6 (CIYVGSSGSTYYASWAKG),
HCDR3 comprises the amino acid sequence according to SEQ ID NO: 7 (GYVDYAGVTYGTDYFNL);
optionally wherein one or more of the CDRs comprises one, two or three amino acid mutations relative to the recited sequences.

In some embodiments, the VL domain comprises light chain complementarity determining regions (LCDRs) 1-3, wherein (according to Kabat numbering):
LCDR1 comprises the amino acid sequence according to SEQ ID NO: 8 (QASEDIYSNLA),
LCDR2 comprises the amino acid sequence according to SEQ ID NO: 9 (GASTLAS), and
LCDR3 comprises the amino acid sequence according to SEQ ID NO: 10 (QGGYYSRSAA);
optionally wherein one or more of the CDRs comprises one, two or three amino acid mutations relative to the recited sequences.

In some embodiments, the VH domain comprises heavy chain complementarity determining regions (HCDRs) 1-3, and the VL domain comprises light chain complementarity determining regions (LCDRs) 1-3, wherein (according to Kabat numbering):
HCDR1 comprises the amino acid sequence according to SEQ ID NO: 5 (SSIYYMC),
HCDR2 comprises the amino acid sequence according to SEQ ID NO: 6 (CIYVGSSGSTYYASWAKG),
HCDR3 comprises the amino acid sequence according to SEQ ID NO: 7 (GYVDYAGVTYGTDYFNL);
LCDR1 comprises the amino acid sequence according to SEQ ID NO: 8 (QASEDIYSNLA),
LCDR2 comprises the amino acid sequence according to SEQ ID NO: 9 (GASTLAS), and
LCDR3 comprises the amino acid sequence according to SEQ ID NO: 10 (QGGYYSRSAA);
optionally wherein one or more of the CDRs comprises one, two or three amino acid mutations relative to the recited sequences.

In some embodiments, the VH domain comprises heavy chain complementarity determining regions (HCDRs) 1-3, wherein (according to Kabat numbering):
HCDR1 comprises the amino acid sequence according to SEQ ID NO: 11 (SSYGVS),
HCDR2 comprises the amino acid sequence according to SEQ ID NO: 12 (YIDPVFDSTYYASWVNG),
HCDR3 comprises the amino acid sequence according to SEQ ID NO: 13 (GGMVIRL);
optionally wherein one or more of the CDRs comprises one, two or three amino acid mutations relative to the recited sequences.

In some embodiments, the VL domain comprises light chain complementarity determining regions (LCDRs) 1-3, wherein (according to Kabat numbering):
LCDR1 comprises the amino acid sequence according to SEQ ID NO: 14 (QASQSISTALA),
LCDR2 comprises the amino acid sequence according to SEQ ID NO: 15 (DASKLAS), and
LCDR3 comprises the amino acid sequence according to SEQ ID NO: 16 (QHGYYSSSTVYNA);
optionally wherein one or more of the CDRs comprises one, two or three amino acid mutations relative to the recited sequences.

In some embodiments, the VH domain comprises heavy chain complementarity determining regions (HCDRs) 1-3, and the VL domain comprises light chain complementarity determining regions (LCDRs) 1-3, wherein (according to Kabat numbering):
HCDR1 comprises the amino acid sequence according to SEQ ID NO: 11 (SSYGVS),
HCDR2 comprises the amino acid sequence according to SEQ ID NO: 12 (YIDPVFDSTYYASWVNG),
HCDR3 comprises the amino acid sequence according to SEQ ID NO: 13 (GGMVIRL);
LCDR1 comprises the amino acid sequence according to SEQ ID NO: 14 (QASQSISTALA),
LCDR2 comprises the amino acid sequence according to SEQ ID NO: 15 (DASKLAS), and
LCDR3 comprises the amino acid sequence according to SEQ ID NO: 16 (QHGYYSSSTVYNA);
optionally wherein one or more of the CDRs comprises one, two or three amino acid mutations relative to the recited sequences.

In some embodiments, the VH domain comprises a sequence having at least 70%, at least 75%, at least 80%, at least 85%, at least 90%, at least 95%, at least 97%, at least 99%, or 100% identity to SEQ ID NO: 27.

In some embodiments, the VL domain comprises a sequence having at least 70%, at least 75%, at least 80%, at least 85%, at least 90%, at least 95%, at least 97%, at least 99%, or 100% identity to SEQ ID NO: 28.

In some embodiments:
(i) the VH domain comprises a sequence having at least 70%, at least 75%, at least 80%, at least 85%, at least 90%, at least 95%, at least 97%, at least 99%, or 100% identity to SEQ ID NO: 27; and
(ii) the VL domain comprises a sequence having at least 70%, at least 75%, at least 80%, at least 85%, at least 90%, at least 95%, at least 97%, at least 99%, or 100% identity to SEQ ID NO: 28.

In some embodiments, the VH domain comprises a sequence having at least 70%, at least 75%, at least 80%, at least 85%, at least 90%, at least 95%, at least 97%, at least 99%, or 100% identity to SEQ ID NO: 29.

In some embodiments, the VL domain comprises a sequence having at least 70%, at least 75%, at least 80%, at least 85%, at least 90%, at least 95%, at least 97%, at least 99%, or 100% identity to SEQ ID NO: 30.

In some embodiments:
(i) the VH domain comprises a sequence having at least 70%, at least 75%, at least 80%, at least 85%, at least 90%, at least 95%, at least 97%, at least 99%, or 100% identity to SEQ ID NO: 29; and
(ii) the VL domain comprises a sequence having at least 70%, at least 75%, at least 80%, at least 85%, at least 90%, at least 95%, at least 97%, at least 99%, or 100% identity to SEQ ID NO: 30.

In some embodiments, the antibody or fragment thereof comprises: a constant heavy chain comprising a sequence having at least 70%, at least 75%, at least 80%, at least 85%, at least 90%, at least 95%, at least 97%, at least 99%, or 100% identity to SEQ ID NO: 31.

In some embodiments, the antibody or fragment thereof comprises: a constant light chain comprising a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises:
(i) a constant heavy chain comprising a sequence having at least 70%, at least 75%, at least 80%, at least 85%, at least 90%, at least 95%, at least 97%, at least 99%, or 100% identity to SEQ ID NO: 31; and
(ii) a constant light chain comprising a sequence having at least 70%, at least 75%, at least 80%, at least 85%, at least 90%, at least 95%, at least 97%, at least 99%, or 100% identity to SEQ ID NO: 32.

In some embodiments, the modulator is antibody clone MM0205-8D12, available from Novus Biologicals. In some embodiments, the inhibitor is antibody clone MM0205-8D12, available from Novus Biologicals. In some embodiments, the binding molecule is antibody clone MM0205-8D12, available from Novus Biologicals. In some embodiments, the anti-CFC1 antibody or fragment thereof is antibody clone MM0205-8D12, available from Novus Biologicals.

In some embodiments, the modulator is antibody M09 clone 2G4, available from Novus Biologicals. In some embodiments, the inhibitor is antibody M09 clone 2G4, available from Novus Biologicals In some embodiments, the binding molecule is antibody M09 clone 2G4, available from Novus Biologicals. In some embodiments, the anti-CFC1 antibody or fragment thereof is antibody M09 clone 2G4, available from Novus Biologicals.

The present invention also provides a polynucleotide for use in treating neuroendocrine neoplasms, wherein the polynucleotide comprises a nucleic acid sequence encoding the modulator as defined according to the invention.

In some embodiments, the nucleic acid sequence is an RNA sequence.

The present invention also provides a vector for use in treating neuroendocrine neoplasms, wherein the vector comprises the polynucleotide as defined according to the invention.

The present invention also provides a composition for use in treating neuroendocrine neoplasms, wherein the composition comprises the modulator as defined according to the invention or the polynucleotide as defined according to the invention, or the vector as defined according to the invention.

The present invention also provides a pharmaceutical composition for use in treating neuroendocrine neoplasms, wherein the pharmaceutical composition comprises the modulator as defined according to the invention, or the polynucleotide as defined according to the invention, or the vector as defined according to the invention, or the composition as defined according to the invention; together with a pharmaceutically acceptable carrier, diluent or excipient.

In some embodiments, the pharmaceutical composition further comprising a vesicle, a nanoparticle, a lipid nanoparticle (LNP), a liposome or a polymeric mixture.

In some embodiments, the modulator, the polynucleotide, the vector, or the composition is encapsulated within the vesicle, nanoparticle, LNP, liposome or polymeric mixture.

In some embodiments, the neuroendocrine neoplasm is a tumour or cancer expressing CFC1. In some embodiments, the expression of CFC1 is increased compared to the expression of CFC1 by a non-cancerous tissue.

In some embodiments, the neuroendocrine neoplasm is selected from the list consisting of: a colorectal neuroendocrine tumor, a pancreatic neuroendocrine tumor, a gastroenteropancreatic (GEP) tumour, and a pulmonal neuroendocrine tumor.

In some embodiments, the neuroendocrine neoplasm is not neuroblastoma.

### BRIEF DESCRIPTION OF THE FIGURES

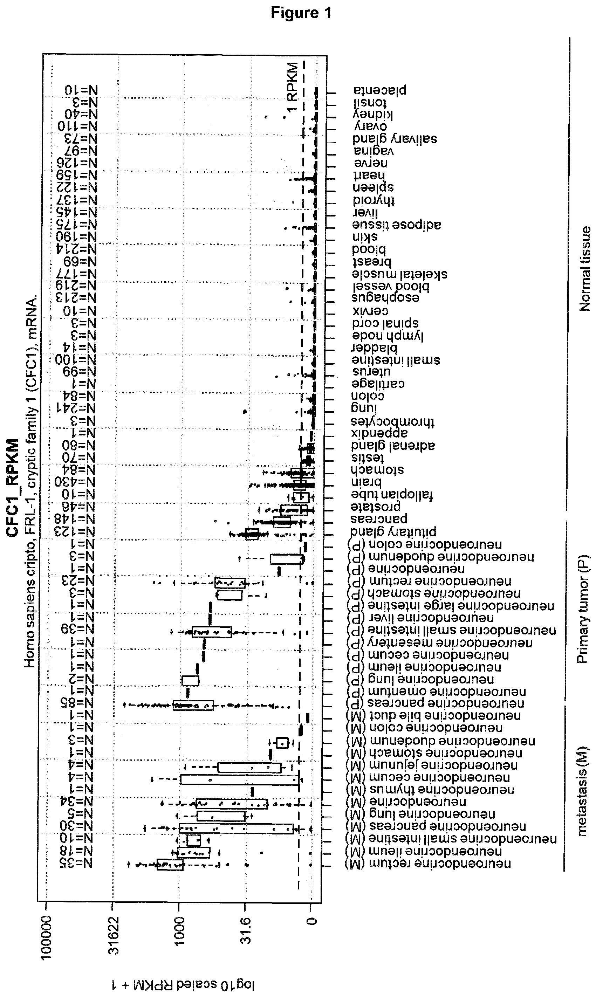

Figure 1 - RNA-Seq expression of CFC1 in NEN primary tumors, NEN derived metastasis and normal tissue. Expression of CFC1 was analyzed using RNA-Seq of primary neuroendocrine neoplasms (P), neuroendocrine derived metastasis (M) and normal tissue samples. Within the boxplot, the lower and upper end of the box correspond to the first and third quartiles and the median value is depicted by a horizontal line in the box. All samples are shown by individual black dots. Target-expressing tumors are defined as having a relative expression level of at least 1 RPKM, indicated by a horizontal dashed line.

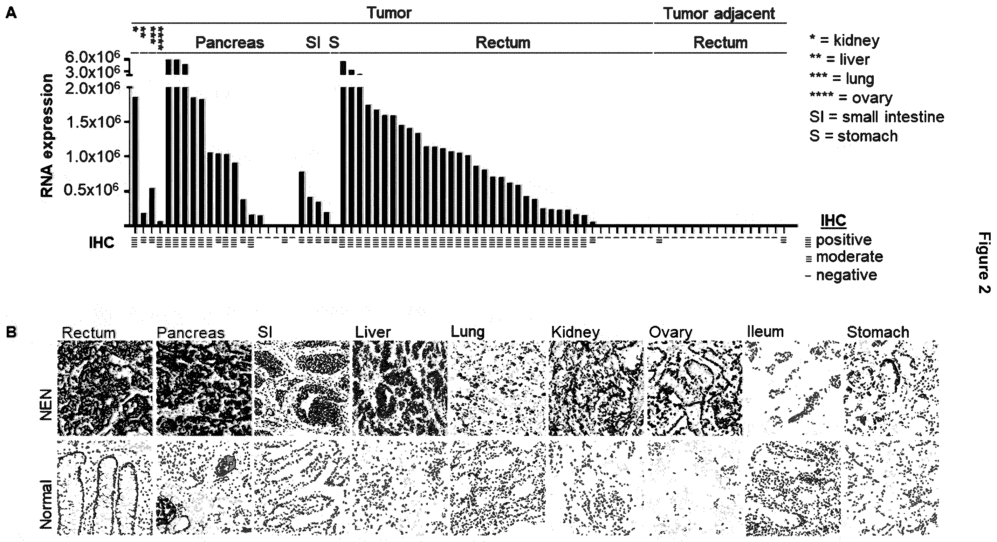

Figure 2 - CFC1 expression in neuroendocrine neoplasms from diverse sites measured by qRT-PCR and IHC. (A) Relative expression of CFC1 was analysed using qRT-PCR of neuroendocrine target-expressing tumors are defined as having a relative expression level of at least 5000 arbitrary units. Corresponding IHC results are indicated as positive, moderate positive or negative at the x-axis. (B) The panel shows selected examples of IHC images for NET samples from diverse origins and their corresponding adjacent tissues.

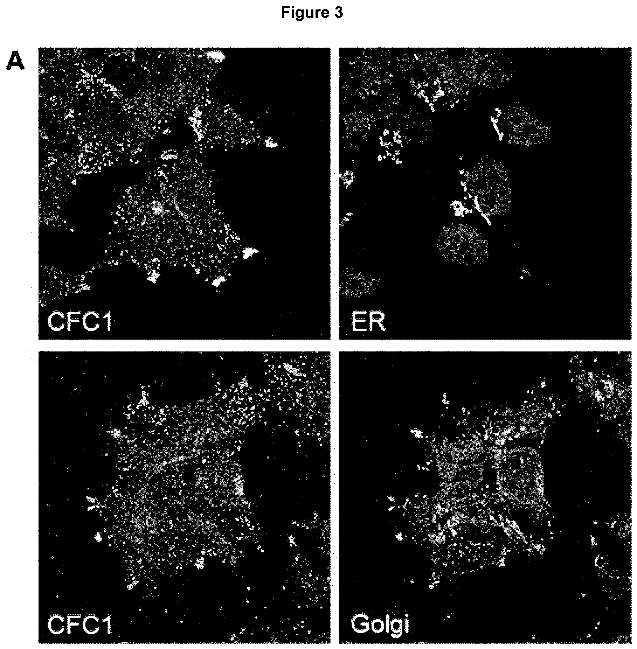

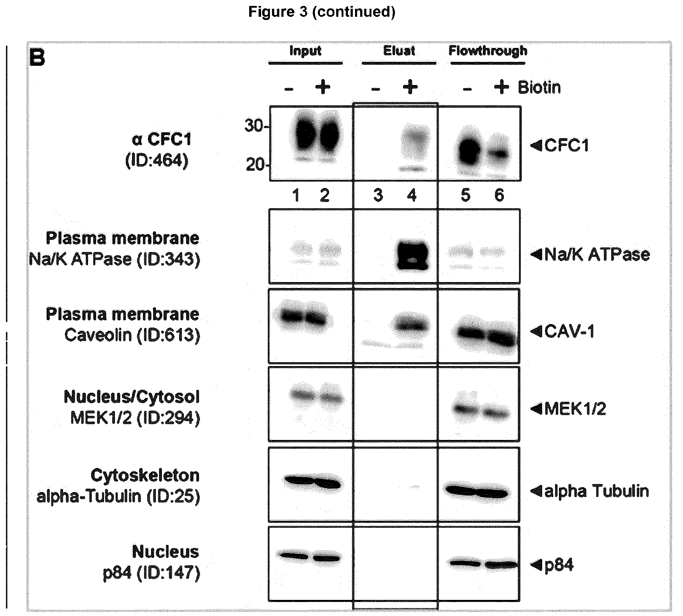

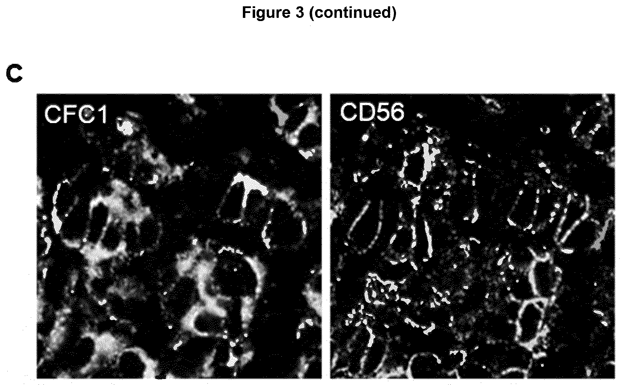

Figure 3 - Sub-cellular localization of CFC1 in NEN cells. (A) Immunofluorescence images showing CFC1 at the membrane, the endoplasmic reticulum (ER) and Golgi apparatus. Nuclear staining was performed using Hoechst. (B) Membrane localization of CFC1 was analyzed using a streptavidin Western blot analysis. The blot includes input, eluate, and flow through from lysed NCI-H810-CFC1 cells incubated with or without biotin. Samples were tested for CFC1 alongside specific markers: Na⁺/K⁺ ATPase and Caveolin-1 (plasma membrane), MEK1/2 (nucleus/cytoskeleton), tubulin (cytoskeleton), and p84 (nucleus). (C) Immunofluorescent staining of CFC1 in rectal NEN tissue. Human tissue sample was stained with antibodies against CFC1 and CD56.

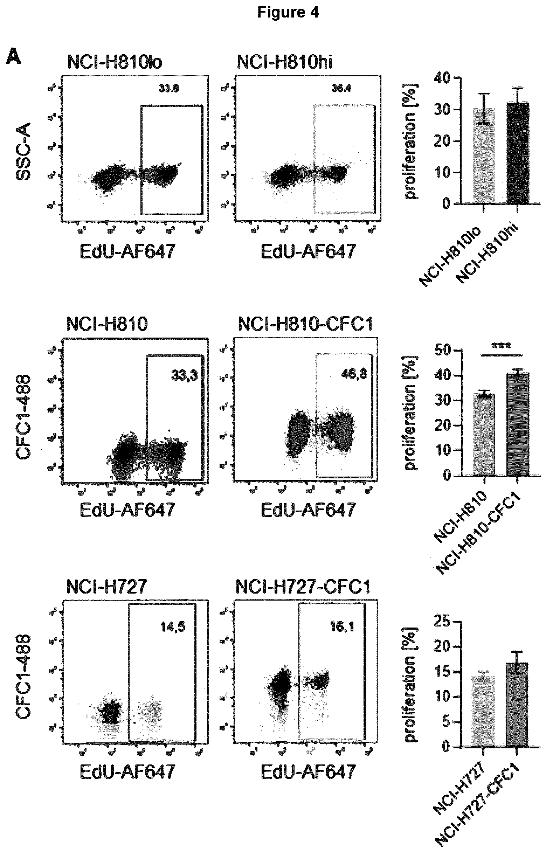

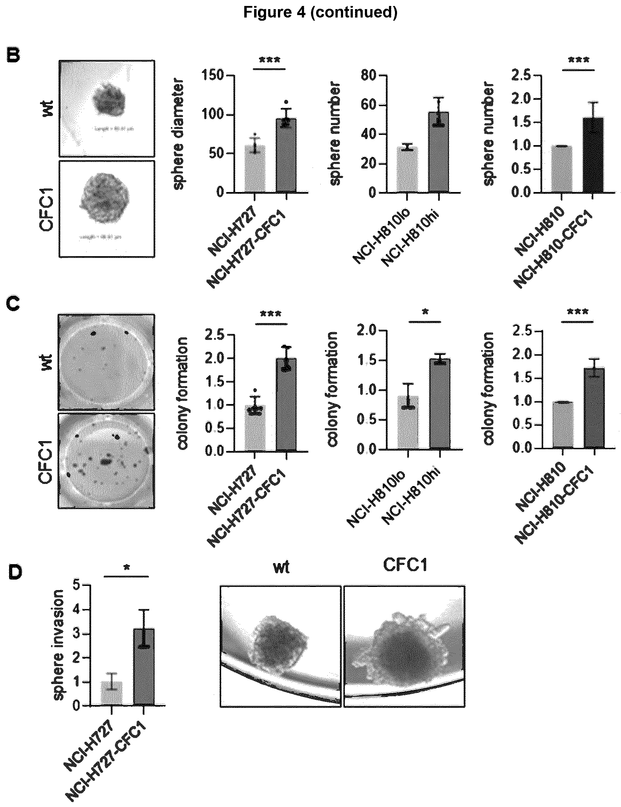

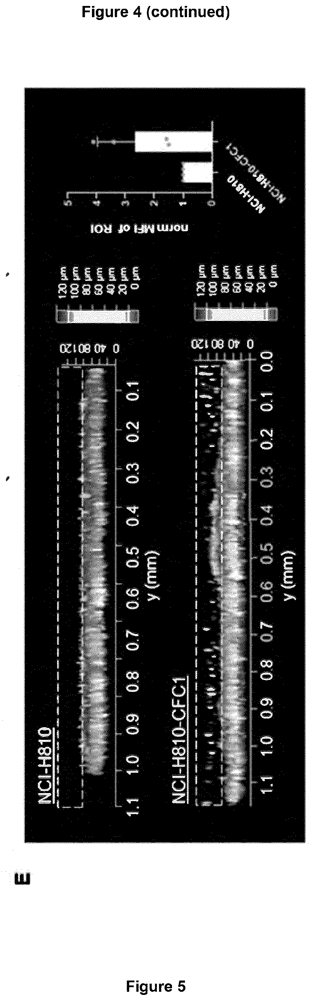

Figure 4 - Functional characterization of CFC1 in NEN cell lines. (A) The effect of CFC1 on proliferation of NEN cell lines was assessed using an EdU incorporation assay. (B, C) The role of CFC1 in promoting cancer stemness was evaluated using (B) a sphere formation assay and (C) a colony formation assay (CFA). (B) A total of 100-200 NEN cells were seeded into a 96-well ultra-low attachment plate. After three weeks of cultivation, spheroid diameters were measured under the microscope. (C) In the CFA, 60-100 NEN cells were seeded into a 24-well plate and cultured for 14 days. Colonies were stained with Crystal violet for evaluation. Representative images from wild type and CFC1-overexpressing cells are shown. (D) Invasion of NCI-H727 cells was assessed using a 3D Matrigel model. Spheroids were embedded in Matrigel, and invasion was measured after ten days by counting the number of invasive spreads under a microscope. (E) Migration of NCl-H810 cells was evaluated using a Migration and Invasion Assay (MANIA). NCI-H810 cells stained with Cell Tracker Orange were covered with Matrigel, and complete media was added. A multi-stack projection from confocal microscopy after two days shows migration depth, color-coded from 0 µm (bottom) to 120 µm (top). Quantification of migration was based on mean fluorescence intensity (MFI) measured from 80-120 µm. Significance in (A-D) was determined using paired two-tiled Student's t-test. Mean ± SD.

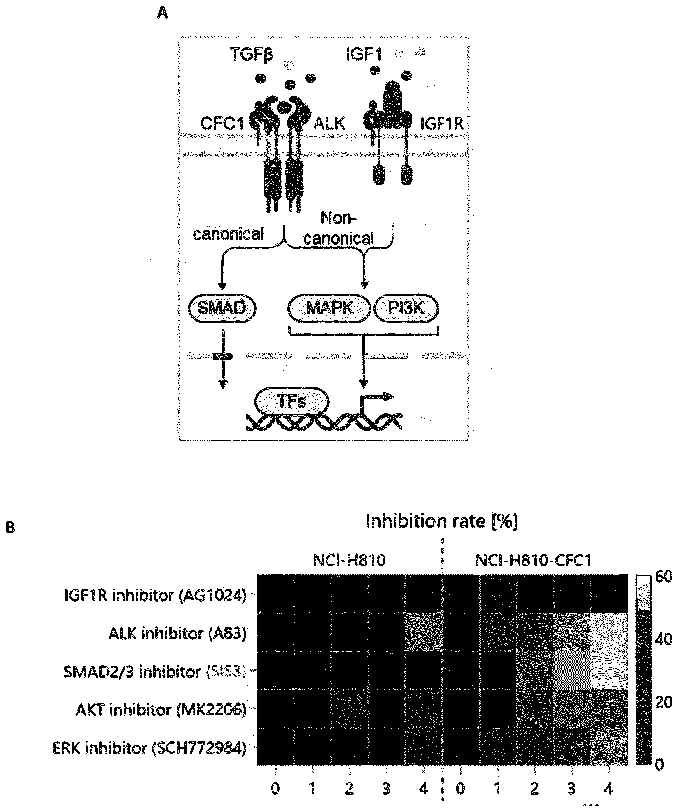

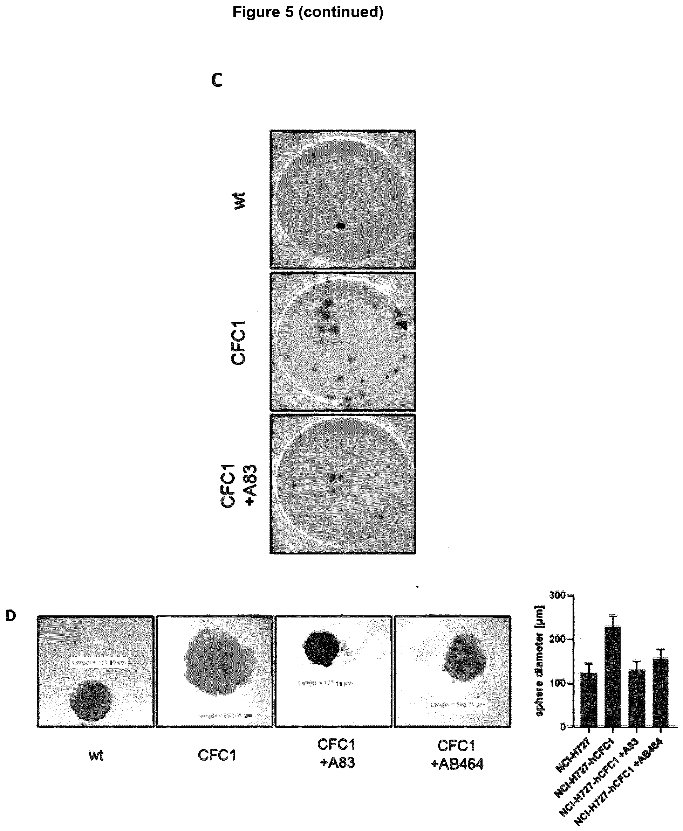

Figure 5 - Involvement of signaling pathways in CFC1-dependent cancer stemness promotion. (A) Schematic representation of signaling pathways tested for their association with CFC1-dependent promotion of cancer stemness. (B) Colonies of the indicated cell lines were treated with inhibitors targeting key signaling molecules at four different concentrations (marked with 1-4). Shown is the inhibition rate of colony formation. (C) Representative microscopic images of colonies treated with the ALK inhibitor A83 or left untreated in NCI-H810 cell lines. (D) Spheroids of NCl-H727-CFC1 cell line were treated with the ALK4/5/7 inhibitor (A83) or CFC1-specific monoclonal antibody (AB464) and compared to NCI-H727 cell line.

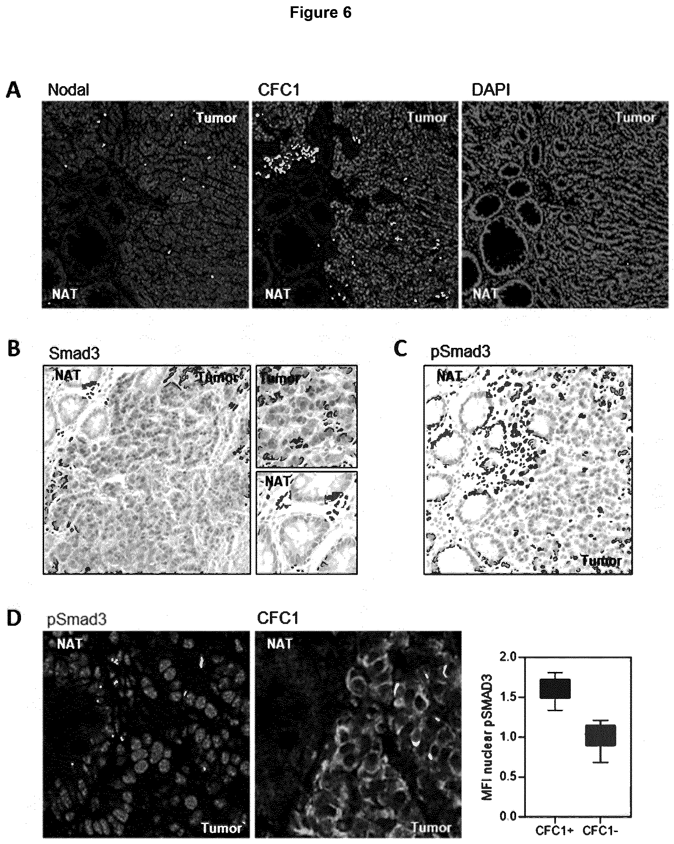

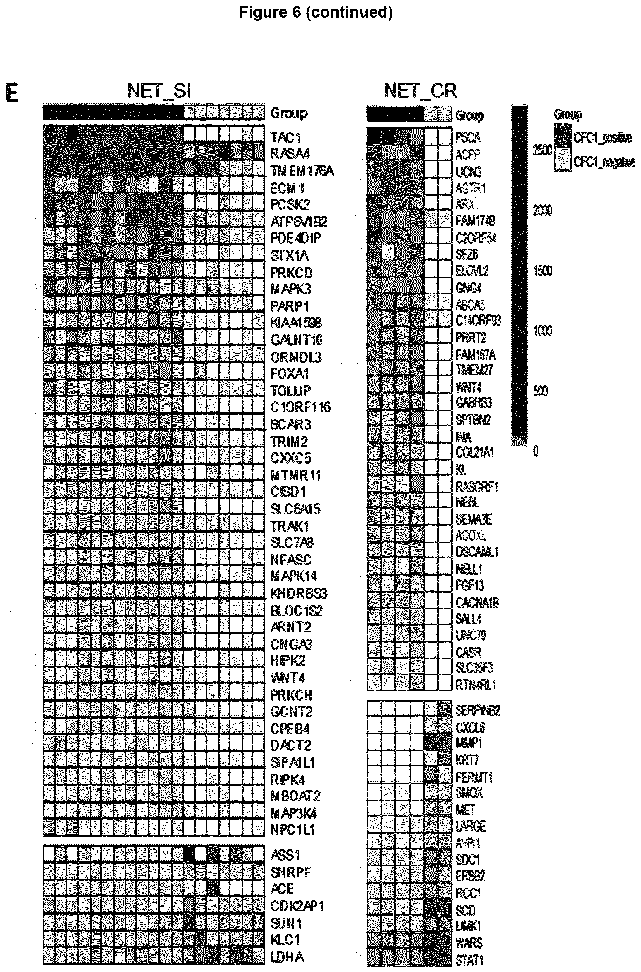

Figure 6 - Assessment of CFC1/SMAD signaling pathways upregulation in human NENs. (A) Representative images of Nodal and CFC1 expression in human NENs compared to adjacent tissue. (B) and (C) Representative images of SMAD3 (B) and phosphorylated SMAD3 (C). (D) Immunofluorescent staining and quantification of pSMAD3 comparing CFC1 positive with CFC1 negative cells in NEN human tumors. Box plots represent the interquartile range (IQR), while whiskers extend to the minimum and maximum values within the data. (E) Top 50 differentially expressed SMAD2/3 target genes in CFC1-positive and CFC1-negative human NENs.

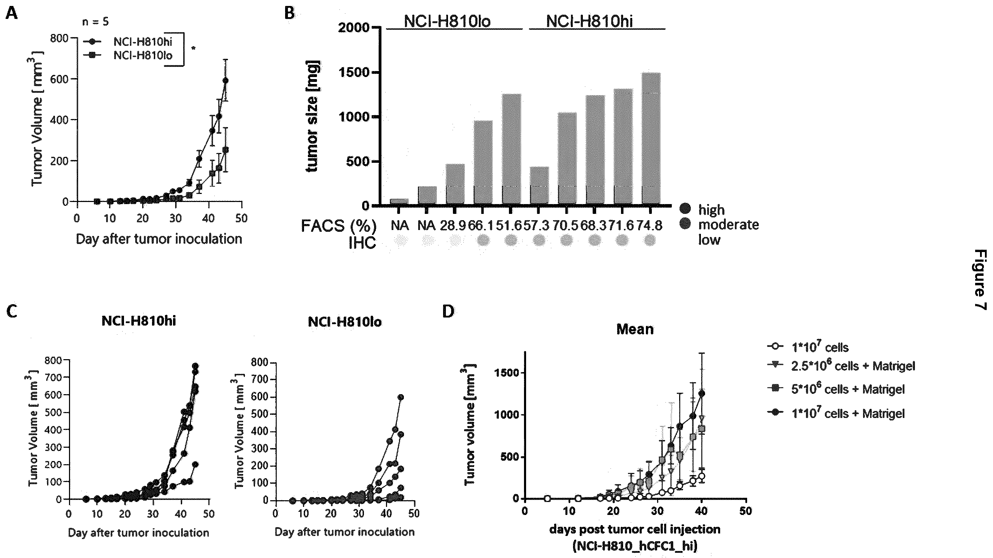

Figure 7 - Impact of CFC1 expression on tumor growth in vivo. (A, C) Mean (A) and individual (C) tumor growth curves of NSG mice (n=5/group) inoculated subcutaneously (s.c.) with 5x106 NCI-H810 tumor cells sorted for low and high CFC1 expression. Significance in (A) was determined using ordinary one-way ANOVA. Mean ± SEM. (B) Bar graph showing CFC1 expression levels measured by flow cytometry (shown in %) and immunohistochemistry in tumors derived from NCI-H810 cells sorted for low high CFC1 expression. (D) Mean tumor growth curves of NSG mice inoculated subcutaneously with different cell numbers, either without Matrigel (1x107) or with Matrigel (2.5x106, 5x106, 1x107), using NCI-H810 tumor cells sorted for high CFC1 expression.

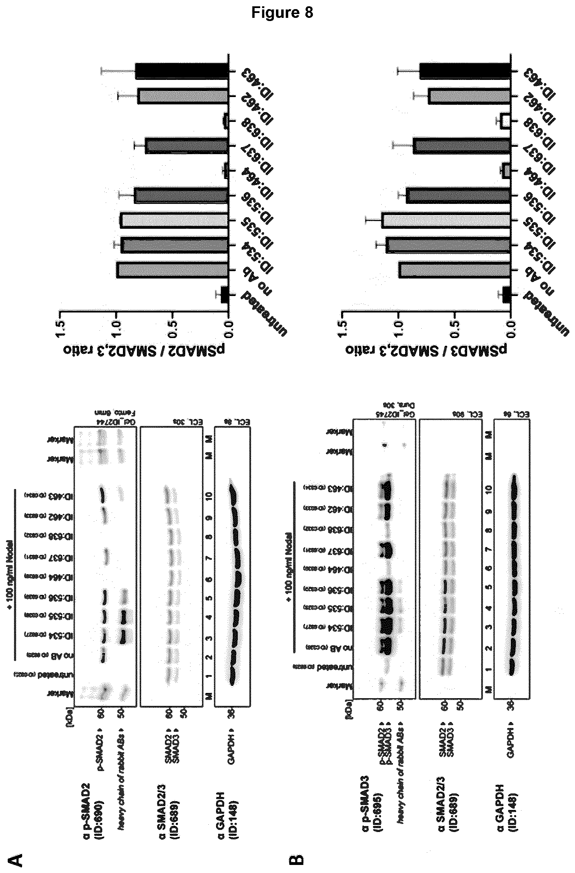

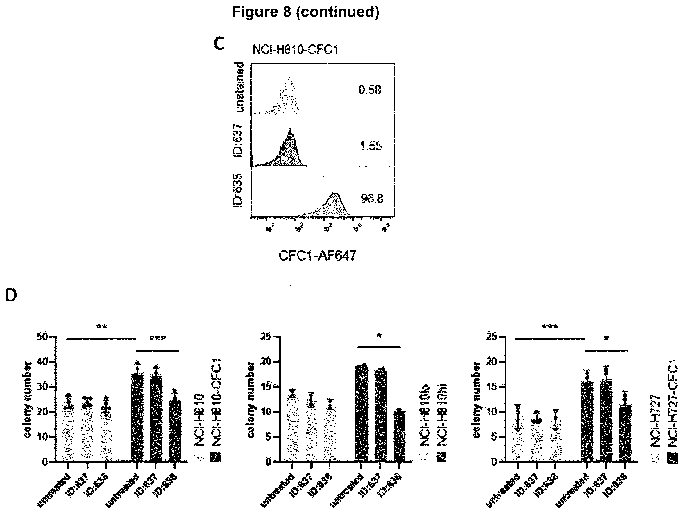

Figure 8 - Effect of commercially available CFC1-specific antibodies on SMAD2/3 signaling induction and cancer stemness promotion. (A, B) NCI-H810-CFC1 cells were treated with 1 µg/mL of commercially available CFC1-specific antibodies. After 30 min, cells were stimulated with Nodal as indicated. 60 min later, cells were lysed and lysates were subjected to Western blot analysis using antibodies against phospho-SMAD2 (A), phospho-SMAD3 (B), SMAD2/3, and GAPDH as loading control (left). Ratio of pSMAD3 and total SMAD2/3 protein was quantified (right). (C) Binding of CFC1-specific antibodies ID:637 and ID:638, as determined with flow cytometry. (D) NEN cells were treated with 1µg/ml of ID:637 (negative control) or ID:638 (positive control) 24 hours after seeding. Colony growth was measured after 14 days and compared to the untreated control. Significance was determined using a paired two-tailed Student's t-test. Mean ± SD.

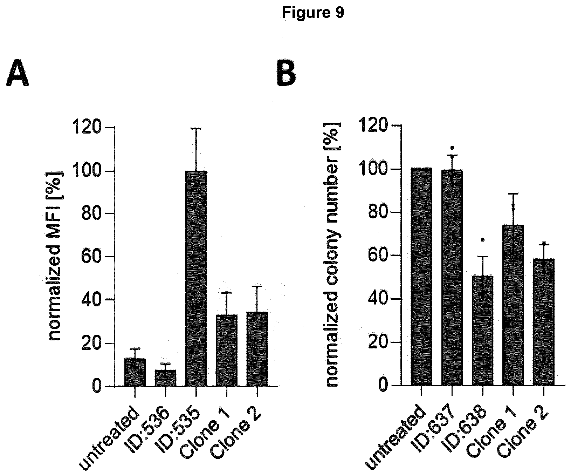

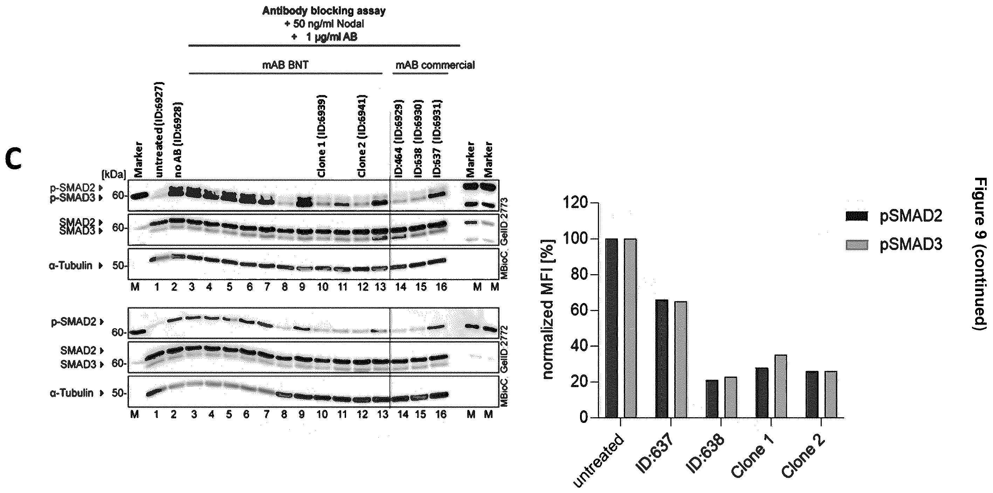

Figure 9 - Effect of CFC1-specific antibodies on SMAD2/3 signaling induction and cancer stemness promotion. (A) NCI-H810-CFC1 cells were treated with 1µg of CFC1-specific antibodies. After 1-2 hours, immunofluorescent staining was performed, and endocytosis was analyzed by microscopy. Antibody uptake was quantified based on mean fluorescence intensity (MFI) (%). (B) NEN cells were treated with 1µg/ml of ID:637 (negative control), ID:638 (positive control), or clone 1 or clone 2 24 hours after seeding. Colony growth was measured after 14 days and compared to the untreated control. Significance was determined using a paired two-tailed Student's t-test. Mean ± SD. (C) NCl-H810-CFC1 cells were treated with 1 µg/mL of CFC1-specific antibodies. After 30 minutes, cells were stimulated with Nodal as indicated. 60 minutes later, cells were lysed and lysates were subjected to Western blot analysis using antibodies against phospho-SMAD2/3, SMAD2/3 and α-Tubulin (loading control) (left). Ratio of pSMAD2/3 and total SMAD2/3 protein was quantified (right).

### DETAILED DESCRIPTION OF THE INVENTION

### CFC1

The antigen Cripto, FRL-1, Cryptic Family 1 (CFC1), also known as Cryptic protein, belongs to the family of epidermal growth factors, which are involved in signaling during embryonal development (Saloman et al. 2000; Shen und Schier 2000). CFC1 protein is important for embryonal left-right patterning and mutations in this gene are associated with defects in organ development (Shen und Schier 2000).

CFC1 is a cell surface receptor that is attached to the cell membrane via a GPI anchor. CFC1 likely functions as co-receptor since it does not have any transmembrane domain that would permit signal transduction. NODAL (Yan et al, 1999; Yan et al, 2002; Fuerer et al, 2014) and GDF1 (growth differentiation factor) (Tanaka, 2007; Fuerer 2014) ligands of the TGF-beta signaling pathway, are the only described ligands for CFC1. Furthermore, CFC1 was previously described as a pancreatic islet marker (Danielsson et al. 2014).

Furthermore, CFC1 has been implicated as a cancer stem cell marker in neuroblastoma (Tsubota und Kadomatsu 2017; Chikaraishi et al. 2017).

The present inventors have surprisingly found that CFC1 is associated with neuroendocrine neoplasms, such as neuroendocrine tumours and neuroendocrine cancer, as CFC1 is expressed, in particular highly expressed, on such neuroendocrine neoplasms.

Without wishing to be bound by theory, CFC1 may enhance or promote the formation of neuroendocrine neoplasms.

An illustrative amino acid sequence of CFC1 is the human CFC1 sequence designated as UniProt: P0CG37.

In some embodiments, CFC1 as described herein is defined according to the UniProt entry: P0CG37.

In some embodiments, CFC1 comprises an amino acid sequence according to SEQ ID NO: 1 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 1. In some embodiments, the CFC1 antigen consists of an amino acid sequence according to SEQ ID NO: 1.

SEQ ID NO: 1
YQREKHNGGREEVTKVATQKHRQSPLNWTSSHFGEVTGSAEGWGPEEPLPYSRAFGEGA
SARPRCCRNGGTCVLGSFCVCPAHFTGRYCEHDQRRSECGALEHGAWTLRACHLCRCIF
GALHCLPLQTPDRCDPKDFLASHAHGPSAGGAPSLLLLLPCALLHRLLRPDAPAHPRSLVPS
VLQRERRPCGRPGLGHRL

In some embodiments, CFC1 comprises an amino acid sequence according to SEQ ID NO: 2 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 2. In some embodiments, the CFC1 antigen consists of an amino acid sequence according to SEQ ID NO: 2.

SEQ ID NO: 2
QREKHNGGREEVTKVATQKHRQSPLNWTSSHFGEVTGSAEGWGPEEPLPYSRAFGEGAS
ARPRCCRN

In some embodiments, CFC1 comprises an amino acid sequence according to SEQ ID NO: 3 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 3. In some embodiments, the CFC1 antigen consists of an amino acid sequence according to SEQ ID NO: 3.

SEQ ID NO: 3
MTWRHHVRLLFTVSLALQIINLGNSYQREKHNGGREEVTKVATQKHRQSPLNWTSSHFGEV
TGSAEGWGPEEPLPYSRAFGEGASARPRCCRNGGTCVLGSFCVCPAHFTGRYCEHDQRR
SECGALEHGAWTLRACHLCRCIFGALHCLPLQTPDRCDPKDFLASHAHGPSAGGAPSLLLL
LPCALLHRLLRPDAPAHPRSLVPSVLQRERRPCGRPGLGHRL

In some embodiments, CFC1 comprises an amino acid sequence according to SEQ ID NO: 4 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 4. In some embodiments, the CFC1 antigen consists of an amino acid sequence according to SEQ ID NO: 4.

SEQ ID NO: 4

YQREKHNGGREEVTKVATQKHRQSPLNWTSSHFGEVTGSAEGWGPEEPLPYSRAFGEGA
SARPRCCRNGGTCVLGSFCVCPAHFTGRYCEHDQRRSECGALEHGAWTLRACHLCRCIF
GALHCLPLQTPDRCDPKDFLASHAHG

### MODULATOR

The present invention provides a modulator of CFC1 for use in treating neuroendocrine neoplasms.

In some embodiments, the modulator antagonizes CFC1 activity. In some embodiments, the modulator inhibits CFC1 signalling. In some embodiments, the modulator inhibits the function of CFC1. In some embodiments, the modulator is capable of recruiting immune cells. In some embodiments, the modulator is capable of targeting immune cells to a tumour or cancer, wherein the tumour or cancer expresses CFC1. In some embodiments, the modulator elicits an immune response. In some embodiments, the modulator elicits an anti-tumoral immune response.

As used herein, the term "modulator" may refer to any therapeutic agent targeting CFC1 on CFC1-expressing tumor cells that:
(i) inhibits CFC1 or CFC1 pathway function(s), and/or
(ii) elicits an anti-tumoral immune response, and/or
(iii) has cytotoxic activity.

Non-limiting examples of such therapeutic agents targeting CFC1-expressing tumor cells include: antibodies, as well as molecules derived from antibodies or immunogenic fragments thereof, molecules blocking or inhibiting the function of CFC1 or of a molecule interacting with CFC1 including small molecules, amino acid or nucleic acid sequences encoding CFC1, antisense nucleic acids hybridisable to a nucleic acid encoding CFC1 or a fragment thereof, siRNAs and vaccines.

In some embodiments, the modulator is an inhibitor of CFC1.

The term "inhibitor" as used herein refers to a molecule which blocks, inhibits, decreases,diminishes or otherwise reduces the expression or function of CFC1 compared to a reference situation wherein, e.g., no inhibitor has been administered, or a standard of care treatment is applied. The inhibitor may be a direct inhibitor, i.e. directly interacting with a CFC1 protein or nucleic acid encoding a CFC1 protein, or an indirect inhibitor, i.e., exerting its inhibitor function via third molecules or mechanisms.

In some embodiments, the modulator is a binding molecule comprising a CFC1 binding domain.

It will be understood that an inhibitor of CFC1 may be a binding molecule comprising a CFC1 binding domain.

In some embodiments, the modulator antagonizes CFC1 activity.

In some embodiments, the modulator inhibits CFC1 signalling.

In some embodiments, the modulator inhibits the function of CFC1.

In some embodiments, the modulator is capable of recruiting immune cells.

In some embodiments, the modulator is capable of targeting immune cells to a tumour or cancer, wherein the tumour or cancer expresses CFC1.

In some embodiments, the modulator elicits an immune response.

In some embodiments, the modulator elicits an anti-tumoral immune response.

### BINDING MOLECULE

The present invention provides a binding molecule comprising a CFC1 binding domain for use in treating neuroendocrine neoplasms.

In some embodiments, the binding molecule antagonizes CFC1 activity.

In some embodiments, the binding molecule inhibits CFC1 signalling.

In some embodiments, the binding molecule inhibits the function of CFC1.

In some embodiments, the binding molecule is capable of recruiting immune cells.

In some embodiments, the binding molecule is capable of targeting immune cells to a tumour or cancer, wherein the tumour or cancer expresses CFC1.

In some embodiments, the binding molecule elicits an immune response.

In some embodiments, the binding molecule elicits an anti-tumoral immune response.

In some embodiments, the binding molecule comprises one or more CFC1 binding domain(s).

In some embodiments, the binding molecule comprises one CFC1 binding domain.

In some embodiments, the binding molecule comprises two CFC1 binding domains.

In some embodiments, the CFC1 binding molecule binds to SEQ ID NO: 1 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 1.

In some embodiments, the CFC1 binding molecule binds to SEQ ID NO: 2 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 2.

In some embodiments, the CFC1 binding molecule binds to SEQ ID NO: 3 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 3.

In some embodiments, the CFC1 binding molecule binds to SEQ ID NO: 4 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 4.

As used herein, a "CFC1 binding molecule" comprises a domain that is capable of binding to CFC1 (a CFC1 binding domain).

It will be understood that theCFC1 binding domain is a domain of a molecule that interacts with a target (e.g., a target antigen) such that a stable association is formed. An example of a molecule comprising a CFC1 binding domain is an antibody (or antibody-like molecule) that binds to its cognate antigen. Such binding interaction will be known in the art. The binding interaction may be non-covalent, reversible covalent or irreversible covalent.

In some embodiments, the CFC1 binding domain antagonizes CFC1 activity.

In some embodiments, the CFC1 binding domain inhibits CFC1 signalling.

In some embodiments, the CFC1 binding domain inhibits the function of CFC1.

In some embodiments, the CFC1 binding domain is capable of recruiting immune cells.

In some embodiments, the CFC1 binding domain is capable of targeting immune cells to a tumour or cancer, wherein the tumour or cancer expresses CFC1.

In some embodiments, the CFC1 binding domain elicits an immune response.

In some embodiments, the CFC1 binding domain elicits an anti-tumoral immune response.

### ANTIBODY

In some embodiments, the binding molecule comprises an antibody as described herein. In some embodiments, the binding molecule consists of an antibody as described herein. In some embodiments, the binding molecule is an antibody as described herein.

In some embodiments, the binding molecule comprises an anti-CFC1 antibody or a CFC1-binding antibody fragment thereof.

In some embodiments, the binding molecule consists of an anti-CFC1 antibody or a CFC1-binding antibody fragment thereof.

In some embodiments, the binding molecule is an anti-CFC1 antibody or a CFC1-binding antibody fragment thereof.

In some embodiments, the anti-CFC1 antibody or a CFC1-binding antibody fragment thereof antagonizes CFC1 activity.

In some embodiments, the anti-CFC1 antibody or a CFC1-binding antibody fragment thereof inhibits CFC1 signalling.

In some embodiments, the anti-CFC1 antibody or a CFC1-binding antibody fragment thereof inhibits the function of CFC1.

In some embodiments, the anti-CFC1 antibody or a CFC1-binding antibody fragment thereof is capable of recruiting immune cells.

In some embodiments, the anti-CFC1 antibody or a CFC1-binding antibody fragment thereof is capable of targeting immune cells to a tumour or cancer, wherein the tumour or cancer expresses CFC1.

In some embodiments, the anti-CFC1 antibody or a CFC1-binding antibody fragment elicits an immune response.

In some embodiments, the anti-CFC1 antibody or a CFC1-binding antibody fragment elicits an anti-tumoral immune response.

It will be understood that the anti-CFC1 antibody or the CFC1-binding antibody fragment thereof may comprise a CFC1 binding domain as described herein.

As used herein, the term "antibody" is well-known in the art and typically means a protein or polypeptide having an antigen-binding domain comprising at least one complementarity determining region(s) (CDR(s)). It will be understood that an antibody may comprises a heavy chain and a light chain.

It will be understood that a fragment of an antibody comprises an amino acid sequence that is shorter than the full-length sequence of an antibody, but retains the biological activity (e.g., binding specificity and/or affinity) of the full-length antibody.

In some embodiments, the antibody or fragment thereof comprises 6 CDRs. It will be understood that such an antibody may be a classical antibody molecule.

In some embodiments, the antibody or fragment thereof comprises 6 CDRs.

In some embodiments, the antibody or fragment thereof comprises a heavy chain variable (VH) domain. In other words, the binding molecule according to the invention may comprise a VH domain.

In some embodiments, the antibody or fragment thereof comprises a light chain variable (VL) domain. In other words, the binding molecule according to the invention may comprise a VL domain.

In some embodiments, the antibody or fragment thereof comprises a VH domain and VL domain. In other words, the binding molecule according to the invention may comprise a VH domain and a VL domain.

It will be understood that the VH domain and/or VL domain are capable of binding to CFC1. It will also be understood that the VH domain and VL domain may form the CFC1 binding domain as described herein.

In some embodiments, the VH domain comprises one or more complementarity determining regions (CDRs). In some embodiments, the VH domain comprises one, two or three CDRs. In some embodiments, the VH domain comprises three CDRs. It will be understood that CDRs of the VH domain may be termed HCDRs. It will also be understood that each of the three CDRs of the VH domain may be termed HCDR1, HCDR2 and HCDR3 respectively.

In some embodiments, the VL domain comprises one or more CDRs. In some embodiments, the VL domain comprises one, two or three CDRs. In some embodiments, the VL domain comprises three CDRs. It will be understood that CDRs of the VL domain may be termed LCDRs. It will also be understood that each of the three CDRs of the VL domain may be termed LCDR1, LCDR2 and LCDR3 respectively.

The terms "heavy chain variable region" or "VH" may refer to the portion of the heavy chain of an antigen-binding molecule, or antibody, that typically contains three CDRs, where each CDR is positioned between stretches of amino acids known as framework regions that form a scaffold to support the CDRs.

The terms "light chain variable region" or "VL" may refer to the fragment of the light chain of an antigen-binding domain, or antibody, that typically contains three CDRs, where each CDR is positioned between stretches of amino acids known as framework regions that form a scaffold to support the CDRs.

The terms "complementarity determining region" or "CDR" refer to a highly variable loop in the variable region which interact with a cognate antigen and is largely responsible for determining the ability of a binding molecule (e.g., an antibody) to bind to an antigen and for determining binding affinity. The CDRs within a variable region are typically numbered from the amino to the carboxy terminus, with CDR1 being closest to the amino terminus of the variable region and CDR3 being closest to the carboxy terminus of the variable region.

The CDRs may be determined according to the Kabat definition and/or the IMGT definition, both of which are well-known in the art.

It will be understood that a variant of a VH domain as described herein may have an equivalent function to a full length VH domain amino acid sequence as described herein and may suitably maintain the capability to bind to CFC1.

It will be understood that a variant of a VL domain as described herein may have an equivalent function to a full length VL domain amino acid sequence as described herein and may suitably maintain the capability to bind to CFC1.

In some embodiments, the binding molecule comprises an immunoglobulin constant region.

In some embodiments, the antibody or fragment thereof according to the invention comprises an immunoglobulin constant region and the VH domain described above and the VL domain described above.

It will be understood that a VH domain and a VL domain may together form a variable region. Thus, the antibody or fragment thereof according to the invention may comprise an immunoglobulin constant region and a variable region.

In some embodiments, the immunoglobulin constant region comprises one or more immunoglobulin constant domain(s).

In some embodiments, the immunoglobulin constant domain is selected from the list consisting of: a constant heavy 1 (CH1) domain, a constant heavy 2 (CH2) domain, and a constant heavy 3 (CH3) domain.

It will be understood that an immunoglobulin constant region may comprise an Fc domain, such as an Fc domain as described herein.

In some embodiments, the antibody or fragment thereof according to the invention comprises an immunoglobulin constant region comprising an Fc domain. In some embodiments, the antibody or fragment thereof according to the invention comprises an immunoglobulin constant region comprising an Fc domain and the VH domain described above and the VL domain described above.

It will be understood that the Fc domain may interact with Fc receptors presented on the surface of a cell. The Fc receptors may be Fc gamma receptors.

In some embodiments, the Fc domain of the antibody or fragment thereof according to the invention is capable of interacting with Fc receptors and/or be able to recruit immune cells.

In some embodiments, the Fc domain of the antibody or fragment thereof according to the invention binds to one or more Fc receptors. In some embodiments, the Fc domain of the antibody or fragment thereof according to the invention binds to one or more Fc gamma receptors.

In some embodiments, the Fc domain of the antibody or fragment thereof according to the invention is capable of binding to immune cells. In some embodiments, the Fc domain of the antibody or fragment thereof according to the invention is capable of recruiting immune cells.

In some embodiments, the immune cells are cytotoxic immune cells. In some embodiments, the immune cells are capable of killing target cells. In some embodiments, the cytotoxic immune cells are selected from the list consisting of: NK cells, macrophages, neutrophils and eosinophils.

In some embodiments, the antibody or fragment thereof according to the invention comprises a heavy chain constant domain. In some embodiments, the heavy chain constant domain is a heavy chain IgG constant domain. In some embodiments, the heavy chain IgG constant domain is a heavy chain IgG1 constant domain. In some embodiments, the heavy chain IgG constant domain is a heavy chain IgG2 constant domain. In some embodiments, the heavy chain IgG constant domain is a heavy chain IgG3 constant domain. In some embodiments, the heavy chain IgG constant domain is a heavy chain IgG4 constant domain.

In some embodiments, the antibody or fragment thereof according to the invention comprises a light chain constant domain. In some embodiments, the light chain constant domain is a light chain kappa constant domain. In some embodiments, the light chain constant domain is a light chain lambda constant domain.

In some embodiments, the antibody is antibody clone MM0205-8D12, available from Novus Biologicals.

In some embodiments, the antibody is antibody M09 clone 2G4, available from Novus Biologicals.

In some embodiments, the antibody or fragment thereof comprises a heavy chain variable (VH) domain; wherein the VH domain comprises heavy chain complementarity determining regions (HCDRs) 1-3, wherein (according to Kabat numbering):
HCDR1 comprises the amino acid sequence according to SEQ ID NO: 5 (SSIYYMC),
HCDR2 comprises the amino acid sequence according to SEQ ID NO: 6 (CIYVGSSGSTYYASWAKG), and
HCDR3 comprises the amino acid sequence according to SEQ ID NO: 7 (GYVDYAGVTYGTDYFNL);
optionally wherein one or more of the CDRs comprises one, two or three amino acid mutations relative to the recited sequences.

In some embodiments, the antibody or fragment thereof comprises a light chain variable (VL) domain; wherein the VL domain comprises light chain complementarity determining regions (LCDRs) 1-3, wherein (according to Kabat numbering):
LCDR1 comprises the amino acid sequence according to SEQ ID NO: 8 (QASEDIYSNLA),
LCDR2 comprises the amino acid sequence according to SEQ ID NO: 9 (GASTLAS), and
LCDR3 comprises the amino acid sequence according to SEQ ID NO: 10 (QGGYYSRSAA);
optionally wherein one or more of the CDRs comprises one, two or three amino acid mutations relative to the recited sequences.

In some embodiments, the antibody or fragment thereof comprises a heavy chain variable (VH) domain and a light chain variable (VL) domain; wherein the VH domain comprises heavy chain complementarity determining regions (HCDRs) 1-3, and the VL domain comprises light chain complementarity determining regions (LCDRs) 1-3, wherein (according to Kabat numbering):
HCDR1 comprises the amino acid sequence according to SEQ ID NO: 5 (SSIYYMC),
HCDR2 comprises the amino acid sequence according to SEQ ID NO: 6 (CIYVGSSGSTYYASWAKG),
HCDR3 comprises the amino acid sequence according to SEQ ID NO: 7 (GYVDYAGVTYGTDYFNL),
LCDR1 comprises the amino acid sequence according to SEQ ID NO: 8 (QASEDIYSNLA),
LCDR2 comprises the amino acid sequence according to SEQ ID NO: 9 (GASTLAS), and
LCDR3 comprises the amino acid sequence according to SEQ ID NO: 10 (QGGYYSRSAA);
optionally wherein one or more of the CDRs comprises one, two or three amino acid mutations relative to the recited sequences.

In some embodiments, the antibody or fragment thereof comprises a heavy chain variable (VH) domain; wherein the VH domain comprises heavy chain complementarity determining regions (HCDRs) 1-3, wherein (according to Kabat numbering):
HCDR1 comprises the amino acid sequence according to SEQ ID NO: 11 (SSYGVS),
HCDR2 comprises the amino acid sequence according to SEQ ID NO: 12 (YIDPVFDSTYYASWVNG), and
HCDR3 comprises the amino acid sequence according to SEQ ID NO: 13 (GGMVIRL);
optionally wherein one or more of the CDRs comprises one, two or three amino acid mutations relative to the recited sequences.

In some embodiments, the antibody or fragment thereof comprises a light chain variable (VL) domain; wherein the VL domain comprises light chain complementarity determining regions (LCDRs) 1-3, wherein (according to Kabat numbering):
LCDR1 comprises the amino acid sequence according to SEQ ID NO: 14 (QASQSISTALA),
LCDR2 comprises the amino acid sequence according to SEQ ID NO: 15 (DASKLAS), and
LCDR3 comprises the amino acid sequence according to SEQ ID NO: 16 (QHGYYSSSTVYNA);
optionally wherein one or more of the CDRs comprises one, two or three amino acid mutations relative to the recited sequences.

In some embodiments, the antibody or fragment thereof comprises a heavy chain variable (VH) domain and a light chain variable (VL) domain; wherein the VH domain comprises heavy chain complementarity determining regions (HCDRs) 1-3, and the VL domain comprises light chain complementarity determining regions (LCDRs) 1-3, wherein (according to Kabat numbering):
HCDR1 comprises the amino acid sequence according to SEQ ID NO: 11 (SSYGVS),
HCDR2 comprises the amino acid sequence according to SEQ ID NO: 12 (YIDPVFDSTYYASWVNG),
HCDR3 comprises the amino acid sequence according to SEQ ID NO: 13 (GGMVIRL), LCDR1 comprises the amino acid sequence according to SEQ ID NO: 14 (QASQSISTALA),
LCDR2 comprises the amino acid sequence according to SEQ ID NO: 15 (DASKLAS), and
LCDR3 comprises the amino acid sequence according to SEQ ID NO: 16 (QHGYYSSSTVYNA);
optionally wherein one or more of the CDRs comprises one, two or three amino acid mutations relative to the recited sequences.

In some embodiments, the antibody or fragment thereof comprises a heavy chain variable (VH) domain; wherein the VH domain comprises heavy chain complementarity determining regions (HCDRs) 1-3, wherein (according to IMGT numbering):
HCDR1 comprises the amino acid sequence according to SEQ ID NO: 17 (GFSFSSIYY),
HCDR2 comprises the amino acid sequence according to SEQ ID NO: 18 (IYVGSSGST), and
HCDR3 comprises the amino acid sequence according to SEQ ID NO: 19 (ARGYVDYAGVTYGTDYFNL);
optionally wherein one or more of the CDRs comprises one, two or three amino acid mutations relative to the recited sequences.

In some embodiments, the antibody or fragment thereof comprises a light chain variable (VL) domain; wherein the VL domain comprises light chain complementarity determining regions (LCDRs) 1-3, wherein (according to IMGT numbering):
LCDR1 comprises the amino acid sequence according to SEQ ID NO: 20 (EDIYSN),
LCDR2 comprises the amino acid sequence GAS, and
LCDR3 comprises the amino acid sequence according to SEQ ID NO: 21 (QGGYYSRSAA);
optionally wherein one or more of the CDRs comprises one, two or three amino acid mutations relative to the recited sequences.

In some embodiments, the antibody or fragment thereof comprises a heavy chain variable (VH) domain and a light chain variable (VL) domain; wherein the VH domain comprises heavy chain complementarity determining regions (HCDRs) 1-3, and the VL domain comprises light chain complementarity determining regions (LCDRs) 1-3, wherein (according to IMGT numbering):
HCDR1 comprises the amino acid sequence according to SEQ ID NO: 17 (GFSFSSIYY),
HCDR2 comprises the amino acid sequence according to SEQ ID NO: 18 (IYVGSSGST),
HCDR3 comprises the amino acid sequence according to SEQ ID NO: 19 (ARGYVDYAGVTYGTDYFNL),
LCDR1 comprises the amino acid sequence according to SEQ ID NO: 20 (EDIYSN), LCDR2 comprises the amino acid sequence GAS, and
LCDR3 comprises the amino acid sequence according to SEQ ID NO: 21 (QGGYYSRSAA);
optionally wherein one or more of the CDRs comprises one, two or three amino acid mutations relative to the recited sequences.

In some embodiments, the antibody or fragment thereof comprises a heavy chain variable (VH) domain; wherein the VH domain comprises heavy chain complementarity determining regions (HCDRs) 1-3, wherein (according to IMGT numbering):
HCDR1 comprises the amino acid sequence according to SEQ ID NO: 22 (GFDFSSYG),
HCDR2 comprises the amino acid sequence according to SEQ ID NO: 23 (IDPVFDST), and
HCDR3 comprises the amino acid sequence according to SEQ ID NO: 24 (VQGGMVIRL);
optionally wherein one or more of the CDRs comprises one, two or three amino acid mutations relative to the recited sequences.

In some embodiments, the antibody or fragment thereof comprises a light chain variable (VL) domain; the VL domain comprises light chain complementarity determining regions (LCDRs) 1-3, wherein (according to IMGT numbering):
LCDR1 comprises the amino acid sequence according to SEQ ID NO: 25 (QSISTA), LCDR2 comprises the amino acid sequence DAS, and
LCDR3 comprises the amino acid sequence according to SEQ ID NO: 26 (QHGYYSSSTVYNA);
optionally wherein one or more of the CDRs comprises one, two or three amino acid mutations relative to the recited sequences.

In some embodiments, the antibody or fragment thereof comprises a heavy chain variable (VH) domain and a light chain variable (VL) domain; wherein the VH domain comprises heavy chain complementarity determining regions (HCDRs) 1-3, and the VL domain comprises light chain complementarity determining regions (LCDRs) 1-3, wherein (according to IMGT numbering):
HCDR1 comprises the amino acid sequence according to SEQ ID NO: 22 (GFDFSSYG),
HCDR2 comprises the amino acid sequence according to SEQ ID NO: 23 (IDPVFDST), HCDR3 comprises the amino acid sequence according to SEQ ID NO: 24 (VQGGMVIRL),
LCDR1 comprises the amino acid sequence according to SEQ ID NO: 25 (QSISTA), LCDR2 comprises the amino acid sequence DAS, and
LCDR3 comprises the amino acid sequence according to SEQ ID NO: 26 (QHGYYSSSTVYNA);
optionally wherein one or more of the CDRs comprises one, two or three amino acid mutations relative to the recited sequences.

In some embodiments, the antibody or fragment thereof comprises a VH domain comprising a sequence having at least 70%, at least 75%, at least 80%, at least 85%, at least 90%, at least 95%, at least 97%, at least 99%, or 100% identity to SEQ ID NO: 27.

In some embodiments, the antibody or fragment thereof comprises a VH domain consisting of a sequence having at least 70%, at least 75%, at least 80%, at least 85%, at least 90%, at least 95%, at least 97%, at least 99%, or 100% identity to SEQ ID NO: 27.

In some embodiments, the antibody or fragment thereof comprises a VH domain comprising a sequence as set forth in SEQ ID NO: 27.

In some embodiments, the antibody or fragment thereof comprises a VH domain consisting of a sequence as set forth in SEQ ID NO: 27.

SEQ ID NO: 27

QSLEESGGGLVKPGASLTLTCTASGFSFSSIYYMCWVRQAPGKGLEWIACIYVGSSGSTYY
ASWAKGRFTISKTSSTTVTLQMTSLTAADTATYFCARGYVDYAGVTYGTDYFNLWGPGTLV
TVSS

In some embodiments, the antibody or fragment thereof comprises a **VL** domain comprising a sequence having at least 70%, at least 75%, at least 80%, at least 85%, at least 90%, at least 95%, at least 97%, at least 99%, or 100% identity to SEQ ID NO: 28.

In some embodiments, the antibody or fragment thereof comprises a **VL** domain consisting of a sequence having at least 70%, at least 75%, at least 80%, at least 85%, at least 90%, at least 95%, at least 97%, at least 99%, or 100% identity to SEQ ID NO: 28.

In some embodiments, the antibody or fragment thereof comprises a **VL** domain comprising a sequence as set forth in SEQ ID NO: 28.

In some embodiments, the antibody or fragment thereof comprises a **VL** domain consisting of a sequence as set forth in SEQ ID NO: 28.

SEQ ID NO: 28
DIVMTQTPASVSAAVEGTVTIKCQASEDIYSNLAWYQQKPGQRPKLLIYGASTLASGVPSRF
KGSGSGTEYTLTISDLESADAATYYCQGGYYSRSAAFGGGTEVVVK

In some embodiments, the antibody or fragment thereof comprises:
(i) a **VH** domain comprising a sequence having at least 70%, at least 75%, at least 80%, at least 85%, at least 90%, at least 95%, at least 97%, at least 99%, or 100% identity to SEQ ID NO: 27; and
(ii) a **VL** domain comprising a sequence having at least 70%, at least 75%, at least 80%, at least 85%, at least 90%, at least 95%, at least 97%, at least 99%, or 100% identity to SEQ ID NO: 28.

In some embodiments, the antibody or fragment thereof comprises:
(i) a **VH** domain consisting of a sequence having at least 70%, at least 75%, at least 80%, at least 85%, at least 90%, at least 95%, at least 97%, at least 99%, or 100% identity to SEQ ID NO: 27; and
(ii) a **VL** domain consisting of a sequence having at least 70%, at least 75%, at least 80%, at least 85%, at least 90%, at least 95%, at least 97%, at least 99%, or 100% identity to SEQ ID NO: 28.

In some embodiments, the antibody or fragment thereof comprises:
(i) a **VH** domain comprising a sequence as set forth in SEQ ID NO: 27; and
(ii) a **VL** domain comprising a sequence as set forth in SEQ ID NO: 28.

In some embodiments, the antibody or fragment thereof comprises:
(i) a **VH** domain consisting of a sequence as set forth in SEQ ID NO: 27; and
(ii) a **VL** domain consisting of a sequence as set forth in SEQ ID NO: 28.

In some embodiments, the antibody or fragment thereof comprises a **VH** domain comprising a sequence having at least 70%, at least 75%, at least 80%, at least 85%, at least 90%, at least 95%, at least 97%, at least 99%, or 100% identity to SEQ ID NO: 29.

In some embodiments, the antibody or fragment thereof comprises a **VH** domain consisting of a sequence having at least 70%, at least 75%, at least 80%, at least 85%, at least 90%, at least 95%, at least 97%, at least 99%, or 100% identity to SEQ ID NO: 29.

In some embodiments, the antibody or fragment thereof comprises a **VH** domain comprising a sequence as set forth in SEQ ID NO: 29.

In some embodiments, the antibody or fragment thereof comprises a **VH** domain consisting of a sequence as set forth in SEQ ID NO: 29.

SEQ ID NO: 29
QEQLVESGGGLVQPGGSLKLSCKASGFDFSSYGVSWVRQAPGKGLEWIGYIDPVFDSTYY
ASWVNGRFTISSHNAQNTLYLQLNSLTAADTATYFCVQGGMVIRLWGPGTLVTVSS

In some embodiments, the antibody or fragment thereof comprises a VL domain comprising a sequence having at least 70%, at least 75%, at least 80%, at least 85%, at least 90%, at least 95%, at least 97%, at least 99%, or 100% identity to SEQ ID NO: 30.

In some embodiments, the antibody or fragment thereof comprises a VL domain consisting of a sequence having at least 70%, at least 75%, at least 80%, at least 85%, at least 90%, at least 95%, at least 97%, at least 99%, or 100% identity to SEQ ID NO: 30.

In some embodiments, the antibody or fragment thereof comprises a VL domain comprising a sequence as set forth in SEQ ID NO: 30.

In some embodiments, the antibody or fragment thereof comprises a VL domain consisting of a sequence as set forth in SEQ ID NO: 30.

SEQ ID NO: 30
AVVLTQTPASVSAAVGGTVTINCQASQSISTALAWYQQKPGQRPKLLIYDASKLASGVPSRF
KGSGSGTEFTLTISGVQSDDAATYYCQHGYYSSSTVYNAFGGGTEVVVK

In some embodiments, the antibody or fragment thereof comprises:
(i) a **VH** domain comprising a sequence having at least 70%, at least 75%, at least 80%, at least 85%, at least 90%, at least 95%, at least 97%, at least 99%, or 100% identity to SEQ ID NO: 29; and
(ii) a **VL** domain comprising a sequence having at least 70%, at least 75%, at least 80%, at least 85%, at least 90%, at least 95%, at least 97%, at least 99%, or 100% identity to SEQ ID NO: 30.

In some embodiments, the antibody or fragment thereof comprises:
(i) a **VH** domain consisting of a sequence having at least 70%, at least 75%, at least 80%, at least 85%, at least 90%, at least 95%, at least 97%, at least 99%, or 100% identity to SEQ ID NO: 29; and
(ii) a **VL** domain consisting of a sequence having at least 70%, at least 75%, at least 80%, at least 85%, at least 90%, at least 95%, at least 97%, at least 99%, or 100% identity to SEQ ID NO: 30.

In some embodiments, the antibody or fragment thereof comprises:
(i) a **VH** domain comprising a sequence as set forth in SEQ ID NO: 29; and
(ii) a **VL** domain comprising a sequence as set forth in SEQ ID NO: 30.

In some embodiments, the antibody or fragment thereof comprises:
(i) a **VH** domain consisting of a sequence as set forth in SEQ ID NO: 29; and
(ii) a **VL** domain consisting of a sequence as set forth in SEQ ID NO: 30.

In some embodiments, the antibody or fragment thereof comprises a constant heavy chain comprising a sequence having at least 70%, at least 75%, at least 80%, at least 85%, at least 90%, at least 95%, at least 97%, at least 99%, or 100% identity to SEQ ID NO: 31.

In some embodiments, the antibody or fragment thereof comprises a constant heavy chain consisting of a sequence having at least 70%, at least 75%, at least 80%, at least 85%, at least 90%, at least 95%, at least 97%, at least 99%, or 100% identity to SEQ ID NO: 31.

In some embodiments, the antibody or fragment thereof comprises a constant heavy chain comprising a sequence as set forth in SEQ ID NO: 31.

In some embodiments, the antibody or fragment thereof comprises a constant heavy chain consisting of a sequence as set forth in SEQ ID NO: 31.

SEQ ID NO: 31
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVMHEALHNHYTQKSLSLSPG

In some embodiments, the antibody or fragment thereof comprises a constant light chain comprising a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises a constant light chain consisting of a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises a constant light chain comprising of a sequence as set forth in SEQ ID NO: 32.

In some embodiments, the antibody or fragment thereof comprises a constant light chain consisting of a sequence as set forth in SEQ ID NO: 32.

SEQ ID NO: 32



In some embodiments, the antibody or fragment thereof comprises:
(i) a constant heavy chain comprising a sequence having at least 70%, at least 75%, at least 80%, at least 85%, at least 90%, at least 95%, at least 97%, at least 99%, or 100% identity to SEQ ID NO: 31; and
(ii) a constant light chain comprising a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises:
(i) a constant heavy chain consisting of a sequence having at least 70%, at least 75%, at least 80%, at least 85%, at least 90%, at least 95%, at least 97%, at least 99%, or 100% identity to SEQ ID NO: 31; and
(ii) a constant light chain consisting of a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises:
(i) a constant heavy chain comprising a sequence as set forth in SEQ ID NO: 31; and
(ii) a constant light chain comprising a sequence as set forth in SEQ ID NO: 32.

In some embodiments, the antibody or fragment thereof comprises:
(i) a constant heavy chain consisting of a sequence as set forth in SEQ ID NO: 31; and
(ii) a constant light chain consisting of a sequence as set forth in SEQ ID NO: 32.

In some embodiments, the antibody or fragment thereof comprises:
(i) a VH domain comprising a sequence having at least 70%, at least 75%, at least 80%, at least 85%, at least 90%, at least 95%, at least 97%, at least 99%, or 100% identity to SEQ ID NO: 27;
(ii) a VL domain comprising a sequence having at least 70%, at least 75%, at least 80%, at least 85%, at least 90%, at least 95%, at least 97%, at least 99%, or 100% identity to SEQ ID NO: 28;
(iii) a constant heavy chain comprising a sequence having at least 70%, at least 75%, at least 80%, at least 85%, at least 90%, at least 95%, at least 97%, at least 99%, or 100% identity to SEQ ID NO: 31; and
(iv) a constant light chain comprising a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises:
(i) a VH domain consisting of a sequence having at least 70%, at least 75%, at least 80%, at least 85%, at least 90%, at least 95%, at least 97%, at least 99%, or 100% identity to SEQ ID NO: 27;
(ii) a VL domain consisting of a sequence having at least 70%, at least 75%, at least 80%, at least 85%, at least 90%, at least 95%, at least 97%, at least 99%, or 100% identity to SEQ ID NO: 28;
(iii) a constant heavy chain consisting of a sequence having at least 70%, at least 75%, at least 80%, at least 85%, at least 90%, at least 95%, at least 97%, at least 99%, or 100% identity to SEQ ID NO: 31; and
(iv) a constant light chain consisting of a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises
(i) a VH domain comprising a sequence as set forth in SEQ ID NO: 27;
(ii) a VL domain comprising a sequence as set forth in SEQ ID NO: 28;
(iii) a constant heavy chain comprising a sequence as set forth in SEQ ID NO: 31; and
(iv) a constant light chain comprising a sequence as set forth in SEQ ID NO: 32.

In some embodiments, the antibody or fragment thereof comprises:
(i) a VH domain consisting of a sequence as set forth in SEQ ID NO: 27;
(ii) a VL domain consisting of a sequence as set forth in SEQ ID NO: 28;
(iii) a constant heavy chain consisting of a sequence as set forth in SEQ ID NO: 31; and
(iv) a constant light chain consisting of a sequence as set forth in SEQ ID NO: 32.

In some embodiments, the antibody or fragment thereof comprises:
(i) a VH domain comprising a sequence having at least 70%, at least 75%, at least 80%, at least 85%, at least 90%, at least 95%, at least 97%, at least 99%, or 100% identity to SEQ ID NO: 29;
(ii) a VL domain comprising a sequence having at least 70%, at least 75%, at least 80%, at least 85%, at least 90%, at least 95%, at least 97%, at least 99%, or 100% identity to SEQ ID NO: 30;
(iii) a constant heavy chain comprising a sequence having at least 70%, at least 75%, at least 80%, at least 85%, at least 90%, at least 95%, at least 97%, at least 99%, or 100% identity to SEQ ID NO: 31; and
(iv) a constant light chain comprising a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises:
(i) a VH domain consisting of a sequence having at least 70%, at least 75%, at least 80%, at least 85%, at least 90%, at least 95%, at least 97%, at least 99%, or 100% identity to SEQ ID NO: 29;
(ii) a VL domain consisting of a sequence having at least 70%, at least 75%, at least 80%, at least 85%, at least 90%, at least 95%, at least 97%, at least 99%, or 100% identity to SEQ ID NO: 30;
(iii) a constant heavy chain consisting of a sequence having at least 70%, at least 75%, at least 80%, at least 85%, at least 90%, at least 95%, at least 97%, at least 99%, or 100% identity to SEQ ID NO: 31; and
(iv) a constant light chain consisting of a sequence having at least 70%, at least 75%, at least 80%, at least 85%, at least 90%, at least 95%, at least 97%, at least 99%, or 100% identity to SEQ ID NO: 32.

In some embodiments, the antibody or fragment thereof comprises:
(i) a VH domain comprising a sequence as set forth in SEQ ID NO: 29;
(ii) a VL domain comprising a sequence as set forth in SEQ ID NO: 30;
(iii) a constant heavy chain comprising a sequence as set forth in SEQ ID NO: 31; and
(iv) a constant light chain comprising a sequence as set forth in SEQ ID NO: 32.

In some embodiments, the antibody or fragment thereof comprises:
(i) a VH domain consisting of a sequence as set forth in SEQ ID NO: 29;
(ii) a VL domain consisting of a sequence as set forth in SEQ ID NO: 30;
(iii) a constant heavy chain consisting of a sequence as set forth in SEQ ID NO: 31; and
(iv) a constant light chain consisting of a sequence as set forth in SEQ ID NO: 32.

It will be understood that Clone 1, as used in the Examples and Figures, is represented by SEQ ID NOs: 5-10, 17-21 and GAS (for LCDR2 IMGT numbering), 27, 28, 31, 32.

It will be understood that Clone 2, as used in the Examples and Figures, is represented by SEQ ID NOs: 11-16, 22-26 and DAS (for LCDR2 IMGT numbering), 29, 30, 31, 32.

In some embodiments, the antibody or fragment thereof is selected from the list consisting of: a non-human antibody, a chimeric antibody, a humanised antibody and a fully human antibody.

In some embodiments, the antibody or fragment thereof is humanised.

In some embodiments, the antibody or fragment thereof is chimeric.

In some embodiments, the antibody is a full-length, classical antibody. In some embodiments, the antibody is an IgG, IgM, IgD, IgE or IgA molecule.

In some embodiments, the antibody is an IgG molecule. In some embodiments, the antibody is an IgG1 molecule. In some embodiments, the antibody is an IgG2 molecule. In some embodiments, the antibody is an IgG3 molecule. In some embodiments, the antibody is an IgG4 molecule.

In some embodiments, the antibody is a monoclonal antibody or fragment thereof. In some embodiments, the antibody is a humanised monoclonal antibody or fragment thereof.

Techniques for obtaining antibodies and fragments thereof are well-known in the art. By way of example, it is well-known that antibodies may be obtained by standard techniques comprising immunizing an animal with a target antigen and isolating antibodies from serum.

The term "humanised antibody" typically refers to a genetically engineered antibody generated in a non-human animal that has been engineered to reduce immunogenicity when used in a human whilst retaining antigen specificity. The humanised antibody typically contains human antibody constant domains and non-human variable domains that are modified to contain a high level of sequence homology to human variable domains.

Suitable assays and techniques for measuring/quantifying binding activity of the antibody or fragment thereof according to the invention may include, but are not limited to, ELISA, surface plasmon resonance (SPR), quartz crystal microbalance (QCM), bioluminescence assays and flow cytometry. Other suitable techniques will be known in the art.

In particular, it will be understood that the ability of a binding molecule to bind to its cognate target may be assessed by flow cytometry, where the effectiveness of a binding molecule to bind to a cognate target expressed by a population of cells can be measured in a flow cytometry assay.

### POLYNUCLEOTIDE

The present invention provides a polynucleotide for use in treating neuroendocrine neoplasms, wherein the polynucleotide comprises a nucleic acid sequence encoding the modulator according to the invention.

The present invention provides a polynucleotide for use in treating neuroendocrine neoplasms, wherein the polynucleotide comprises a nucleic acid sequence encoding the binding molecule according to the invention.

The present invention provides a polynucleotide for use in treating neuroendocrine neoplasms, wherein the polynucleotide comprises a nucleic acid sequence encoding the antibody or fragment thereof according to the invention.

As used herein, the terms "nucleotide sequence", and "nucleic acid sequence" are intended to be synonymous with each other. The nucleic acid sequence may be a DNA or an RNA sequence. The nucleic acid sequence may be single-stranded or may be double-stranded. The nucleic acid sequence may be, for example, genomic, recombinant, cDNA or mRNA. The nucleic acid sequence may comprise synthetic nucleotides and/or modified nucleotides. These synthetic nucleotides and/or modified nucleotides may enhance the *in vivo* activity and/or stability of the polynucleotide.

In some embodiments, the, or each, nucleic acid sequence is a DNA sequence.

In some embodiments, the, or each, nucleic acid sequence is a cDNA sequence.

In some embodiments, the, or each, nucleic acid sequence is an RNA sequence.

In some embodiments, the, or each, nucleic acid sequence is an mRNA sequence.

In some embodiments, the, or each RNA sequence is *in vitro* transcribed RNA.

Due to the redundancy of the genetic code, variations in nucleic acid sequences are possible that encode for the same polypeptide. These sequences are encompassed by the present invention. Therefore, multiple polynucleotides are envisaged, each with a different nucleic acid sequence but which encodes a polypeptide according to the invention or a further polypeptide as described herein. It is known in the art how to design and produce such nucleic acid sequences.

In some embodiments, the nucleic acid sequence of the polynucleotide is codon optimised for production in the host cell of choice.

In some embodiments, the nucleic acid sequence of the polynucleotide is operably linked to a sequence such as a control sequence, e.g., a promoter sequence, an enhancer sequence or regulatory sequence, which controls transcription and/or translation. The polynucleotide may be in the form of an expression cassette.

The polynucleotide may be suitable for expression in prokaryotic cells or in eukaryotic cells, such as mammalian cells.

Any promoter may be used in the polynucleotide, such as a strong promoter that is functional in prokaryotic cells or in eukaryotic cells. Suitable promoters will be known in the art. The promoter may be a constitutive promoter. The promoter may be a tissue specific promoter.

### VECTOR

The present invention provides a vector for use in treating neuroendocrine neoplasms, wherein the vector comprises the polynucleotide according to the invention.

In some embodiments, the vector comprises a polynucleotide comprising a nucleic acid sequence which encodes the modulator according to the invention.

In some embodiments, the vector comprises a polynucleotide comprising a nucleic acid sequence which encodes the binding molecule according to the invention.

In some embodiments, the vector comprises a polynucleotide comprising a nucleic acid sequence which encodes the antibody or fragment thereof according to the invention.

The vector may be used to introduce a polynucleotide according to the invention into a cell so that the cell expresses and/or produces the modulator according to the invention, or the binding molecule according to the invention or the antibody or fragment thereof according to the invention.

As used herein, the term "vector" may be considered interchangeable with the term "expression vector" and "expression construct". The vector may be any vector that is suitable for introducing and/or expressing a nucleic acid sequence in a cell. The vector may comprise regulatory sequences, enhancer sequences and/or promoter sequences that promote expression of a nucleic acid sequence in a cell.

The vector according to the invention may be any agent capable of delivering a polynucleotide according to the invention to a cell.

The vector according to the invention may be any agent capable of expressing a polynucleotide according to the invention in a cell.

Examples of suitable vectors include but are not limited to plasmids, cosmids, phages, viruses or artificial chromosomes.

In some embodiments, the vector is a plasmid or a viral vector. In some embodiments, the vector is a retroviral vector or a lentiviral vector.

The vector may be capable of transfecting or transducing a cell.

### COMPOSITION

The present invention provides a composition for use in treating neuroendocrine neoplasms, wherein the composition comprises the modulator according to the invention.

The present invention provides a composition for use in treating neuroendocrine neoplasms, wherein the composition comprises the binding molecule according to the invention.

The present invention provides a composition for use in treating neuroendocrine neoplasms, wherein the composition comprises the antibody or fragment thereof according to the invention.

The present invention provides a composition for use in treating neuroendocrine neoplasms, wherein the composition comprises the polynucleotide according to the invention.

The present invention provides a composition for use in treating neuroendocrine neoplasms, wherein the composition comprises the vector according to the invention.

### PHARMACEUTICAL COMPOSITION

The present invention provides a pharmaceutical composition for use in treating neuroendocrine neoplasms, wherein the pharmaceutical composition comprises the modulator according to the invention; together with a pharmaceutically acceptable carrier, diluent or excipient.

The present invention also provides a pharmaceutical composition for use in treating neuroendocrine neoplasms, wherein the pharmaceutical composition comprises the binding molecule according to the invention; together with a pharmaceutically acceptable carrier, diluent or excipient.

The present invention also provides a pharmaceutical composition for use in treating neuroendocrine neoplasms, wherein the pharmaceutical composition comprises the inhibitor according to the invention; together with a pharmaceutically acceptable carrier, diluent or excipient.

The present invention also provides a pharmaceutical composition for use in treating neuroendocrine neoplasms, wherein the pharmaceutical composition comprises the antibody or fragment thereof according to the invention; together with a pharmaceutically acceptable carrier, diluent or excipient.

The present invention also provides a pharmaceutical composition for use in treating neuroendocrine neoplasms, wherein the pharmaceutical composition comprises the polynucleotide according to the invention; together with a pharmaceutically acceptable carrier, diluent or excipient.

The present invention also provides a pharmaceutical composition for use in treating neuroendocrine neoplasms, wherein the pharmaceutical composition comprises the vector according to the invention; together with a pharmaceutically acceptable carrier, diluent or excipient.

The present invention also provides a pharmaceutical composition for use in treating neuroendocrine neoplasms, wherein the pharmaceutical composition comprises the composition according to the invention; together with a pharmaceutically acceptable carrier, diluent or excipient.

In some embodiments, the pharmaceutical composition described herein further comprises one or more selected from this list consisting of: a pharmaceutically acceptable adjuvant, salt, active polypeptide, buffer, compound, component and active agent.

In some embodiments, the pharmaceutical composition further comprises a vesicle, a nanoparticle, a lipid nanoparticle (LNP), a liposome or a polymeric mixture.

It will be understood that the vesicle, nanoparticle, LNP, liposome or polymeric mixture may enable delivery of the modulator according to the invention, the inhibitor according to the invention, the binding molecule according to the invention, the antibody or fragment thereof according to the invention, the polynucleotide according to the invention, the vector according to the invention, or the composition according to the invention, to a cell.

In some embodiments, the pharmaceutical composition further comprises a vesicle. In some embodiments, the modulator, the inhibitor, the binding molecule, the antibody or fragment thereof, the polynucleotide, the vector, or the composition according to the invention is encapsulated within the vesicle.

In some embodiments, the pharmaceutical composition further comprises a nanoparticle. In some embodiments, the modulator, the inhibitor, the binding molecule, the antibody or fragment thereof, the polynucleotide, the vector, or the composition according to the invention is encapsulated within the nanoparticle.

In some embodiments, the pharmaceutical composition further comprises a lipid nanoparticle (LNP). In some embodiments, the modulator, the inhibitor, the binding molecule, the antibody or fragment thereof, the polynucleotide, the vector, or the composition according to the invention is encapsulated within the LNP.

In some embodiments, the pharmaceutical composition further comprises a liposome. In some embodiments, the modulator, the inhibitor, the binding molecule, the antibody or fragment thereof, the polynucleotide, the vector, or the composition according to the invention is encapsulated within the liposome.

In some embodiments, the pharmaceutical composition further comprises a polymeric mixture. In some embodiments, the modulator, the inhibitor, the binding molecule, the antibody or fragment thereof, the polynucleotide, the vector, or the composition according to the invention is encapsulated within the polymeric mixture.

Compositions typically should be sterile and stable under the conditions of manufacture and storage. The composition according to the invention may be produced using current good manufacturing practices (CGMP).

In some embodiments, the pharmaceutical composition is sterile.

In some embodiments, the pharmaceutical composition is sterile in aqueous buffer suitable for intravenous (IV) administration.

In some embodiments, the pharmaceutical composition may comprise an aqueous diluent or solvent. In some embodiments, the aqueous diluent or solvent may be a phosphate buffered saline solution, such as a sterile phosphate buffered saline solution.

The term "carrier", as used herein, may refer to a diluent, adjuvant, excipient, or vehicle. Such carriers can be sterile liquids, such as saline solutions in water and oils, including those of petroleum, animal, vegetable or synthetic origin, such as peanut oil, soybean oil, mineral oil, sesame oil. A sterile saline solution is a preferred carrier.

The term "nanoparticle" relates to a nano-sized particle, wherein all three external dimensions of the particle are in the nanoscale, i.e., at least about 1 nm and below about 1,000 nm. Preferably, the size of a particle is its diameter.

Saline solutions and aqueous dextrose and glycerol solutions can also be employed as liquid carriers. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

The composition, if desired, can also contain minor amounts of wetting or emulsifying agents, or pH buffering agents. The composition of the invention can be formulated as neutral or salt forms. Salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

In some embodiments, the salt may comprise a metal cation, such as a sodium salt or a potassium salt.

The pharmaceutical composition may be formulated to be suitable for administration to a patient in order to prevent and/or treat disease. Pharmaceutical compositions can be formulated for administration by different routes, for example, for oral, parenteral, topical, inhalative, intravenous, intramuscular, rectal, sublingual, transdermal, subcutaneous, intratumoral application routes, according to their chemical and physical properties.

In some embodiments, the pharmaceutical composition may be in a form suitable for intravenous infusion. In some embodiments, the pharmaceutical composition may be administered intravenously.

### NEUROENDOCRINE NEOPLASMS

The present invention provides the modulator according to the invention, the inhibitor according to the invention, the binding molecule according to the invention, the antibody or fragment thereof according to the invention, the polynucleotide according to the invention, the vector according to the invention, the composition according to the invention, or the pharmaceutical composition according to the invention, for use in treating and/or preventing neuroendocrine neoplasms (NENs).

It will be understood that neuroendocrine neoplasms comprise well-differentiated neuroendocrine tumors (NETs), poorly differentiated neuroendocrine carcinomas (NECs), and mixed neuroendocrine neoplasm (MINEN). It will also be understood that neuroendocrine neoplasms comprise neuroendocrine microadenoma, NET G1-G3, and NEC.

It will also be understood that neuroendocrine neoplasms can be termed "neuroendocrine neoplasia".

Moreover, it is known in the art that neuroendocrine neoplasms/neuroendocrine neoplasia has a very distinct meaning, describing a distinct disease that excludes neuroblastoma (which have a more embryonal differentiation).

It will be understood that the terms "neuroendocrine neoplasms" and "neuroendocrine neoplasm" can be used interchangeably.

It will also be understood that a neoplasm is a type of abnormal tissue growth, wherein the formation of a neoplasm can be termed a neoplasia.

Thus, in the context of the present invention, a neuroendocrine neoplasm (or neuroendocrine neoplasms) may be referred to as neuroendocrine neoplasia.

The present invention provides the modulator according to the invention for use in treating and/or preventing neuroendocrine neoplasms.

The present invention provides the inhibitor according to the invention for use in treating and/or preventing neuroendocrine neoplasms.

The present invention provides the binding molecule according to the invention for use in treating and/or preventing neuroendocrine neoplasms.

The present invention provides the antibody or fragment thereof according to the invention for use in treating and/or preventing neuroendocrine neoplasms.

The present invention provides the polynucleotide according to the invention for use in treating and/or preventing neuroendocrine neoplasms.

The present invention provides the vector according to the invention for use in treating and/or preventing neuroendocrine neoplasms.

The present invention provides the composition according to the invention for use in treating and/or preventing neuroendocrine neoplasms.

The present invention provides the pharmaceutical composition according to the invention for use in treating and/or preventing neuroendocrine neoplasms.

In some embodiments, the use is for treating neuroendocrine neoplasms. In some embodiments, the use is for preventing neuroendocrine neoplasms.

The present invention provides a method for treating and/or preventing neuroendocrine neoplasms which comprises the step of administering the modulator according to the invention, the inhibitor according to the invention, the binding molecule according to the invention, the antibody or fragment thereof according to the invention, the polynucleotide according to the invention, the vector according to the invention, the composition according to the invention, or the pharmaceutical composition according to the invention, to a subject.

In some embodiments, the method is for treating neuroendocrine neoplasms. In some embodiments, the method is for preventing neuroendocrine neoplasms.

The present invention provides a use of the modulator according to the invention, the inhibitor according to the invention, the binding molecule according to the invention, the antibody or fragment thereof according to the invention, the polynucleotide according to the invention, the vector according to the invention, the composition according to the invention, or the pharmaceutical composition according to the invention, in the manufacture of a medicament for treating and/or preventing neuroendocrine neoplasms.

In some embodiments, the medicament is for treating neuroendocrine neoplasms. In some embodiments, the medicament is for preventing neuroendocrine neoplasms.

In some embodiments, the neuroendocrine neoplasm is a tumour or cancer expressing CFC1.

In some embodiments, the expression of CFC1 is increased compared to the expression of CFC1 by a non-cancerous tissue. It will be understood that non-cancerous tissue typically has low expression, or no detectable expression, of CFC1.

In some embodiments, the neuroendocrine neoplasm is a neuroendocrine tumour (NET)

In some embodiments, the neuroendocrine neoplasm is a neuroendocrine carcinoma (NEC).

In some embodiments, the neuroendocrine neoplasm is selected from the list consisting of: a colorectal neuroendocrine tumor, a pancreatic neuroendocrine tumor, a gastroenteropancreatic (GEP) tumour, and a pulmonal neuroendocrine tumor.

In some embodiments, the neuroendocrine neoplasm is a colorectal neuroendocrine tumor.

In some embodiments, the neuroendocrine neoplasm is a pancreatic neuroendocrine tumor.

In some embodiments, the neuroendocrine neoplasm is a gastroenteropancreatic (GEP) tumour.

In some embodiments, the neuroendocrine neoplasm is a pulmonal neuroendocrine tumor.

It will be understood that the terms "tumor" and "tumours" can be used interchangeably.

In some embodiments, the neuroendocrine neoplasm is a metastasis or metastatic cancer.

In some embodiments, the neuroendocrine neoplasm is not neuroblastoma.

In some embodiments, the neuroendocrine neoplasia is not neuroblastoma. In some embodiments, the cancer is not neuroblastoma. In some embodiments, the tumor is not neuroblastoma.

In some embodiments, the modulator according to the invention, the inhibitor according to the invention, the binding molecule according to the invention, the antibody or fragment thereof according to the invention, the polynucleotide according to the invention, the vector according to the invention, the composition according to the invention, or the pharmaceutical composition according to the invention, is administered to a subject systemically.

In some embodiments, the modulator according to the invention, the inhibitor according to the invention, the binding molecule according to the invention, the antibody or fragment thereof according to the invention, the polynucleotide according to the invention, the vector according to the invention, the composition according to the invention, or the pharmaceutical composition according to the invention, is administered intravenously (IV) to a subject.

The term "disease" and "disorder" are used interchangeably herein, referring to an abnormal condition, especially an abnormal medical condition such as an illness or injury, wherein a cell, a tissue, an organ, or an individual is not able to efficiently fulfil its function anymore. Typically, but not necessarily, a disease is associated with specific symptoms or signs indicating the presence of such disease. The presence of such symptoms or signs may thus, be indicative for a cell, a tissue, an organ, or an individual suffering from a disease. An alteration of these symptoms or signs may be indicative for the progression of such a disease.

A progression of a disease is typically characterised by an increase or decrease of such symptoms or signs which may indicate a "worsening" or "bettering" of the disease. The "worsening" of a disease is characterised by a decreasing ability of a cell, tissue, organ or individual/patient to fulfil its function efficiently, whereas the "bettering" of a disease is typically characterised by an increase in the ability of a cell, tissue, an organ or an individual/patient to fulfil its function efficiently.

The terms "treat", "treatment" and "treating" refers to lessening, reducing or improving at least one symptom associated with an existing disease or condition and/or to slow down, reduce or block the progression of the disease or condition and/or to delay or prevent the onset of symptoms (such as further symptoms) of the disease or condition.

The terms "prevent", "prevention" and "preventing" refers to preventing the onset of symptoms of a disease or condition, and as such encompasses prophylactic treatment.

The medical uses and methods of treatment described herein may be used in combination with additional treatments, therapies and/or medicaments.

### SUBJECT

In some embodiments, the subject is a mammal.

In some embodiments, the subject is a human.

In some embodiments, the subject is a non-human mammal, including for example, a dog, a cat, a horse, a cow, a sheep or a pig.

As used herein, the terms "patient" and "subject" may be used interchangeably.

In some embodiments, the subject is a patient. In some embodiments, the subject is a human patient.

In some embodiments, the subject is a human male. In some embodiments, the subject is a human female.

### IDENTITY

The terms "identity" and "% sequence identity" as used herein, may refer to the proportion of amino acids (expressed in percent) of an amino acid sequence in a peptide or protein, which across the amino acid sequence, are identical to a reference sequence.

The percentage of identity is thus calculated by counting the number of aligned amino acids that are identical (a Match) between two sequences (in the amino acids sequence of the peptide or protein of the invention and in the reference sequence), dividing that number by the total number of amino acids in the aligned region and multiplying by 100.

Therefore, Percentage of Identity = (Matches divided by Length of the aligned region) multiplied by 100.

Insertions and deletions are not allowed in the calculation the percentage of identity of an amino acid sequence.

Any method of calculating percentage identity is permitted within the scope of the invention.

### GENERAL TERMS AND DEFINITIONS

This disclosure is not limited by the exemplary methods and materials disclosed herein, and any methods and materials similar or equivalent to those described herein can be used in the practice or testing of embodiments of this disclosure.

The term "polypeptide" is used in the conventional sense to mean a series of amino acids, typically L-amino acids, connected one to the other, typically by peptide bonds between the α-amino and carboxyl groups of adjacent amino acids.

The term "polypeptide" is used interchangeably with the terms "amino acid sequence", "peptide" and/or "protein".

The term "residues" is used to refer to amino acids in an amino acid sequence.

Numeric ranges are inclusive of the numbers defining the range. Unless otherwise indicated, any nucleic acid sequences are written left to right in 5' to 3' orientation; amino acid sequences are written left to right in amino to carboxy orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the terms "consisting of' or "consisting essentially of".

As used herein, the term "variant" is synonymous with the term "mutant" and refers to an amino acid sequence or a nucleic acid sequence that differs in comparison to the corresponding wild-type sequence. The term "wild-type" is used to mean a protein comprising an amino acid sequence or a polynucleotide comprising an amino acid sequence, which is identical to the native protein or native polynucleotide (e.g. gene) respectively. The variant may have an equivalent function to the amino acid sequences or nucleic acid sequences described herein, but may include one or more amino acid or nucleic acid (respectively) substitutions, insertions or deletions. Amino acid substitutions, insertions and/or deletions may be considered as mutations. Nucleic acid substitutions, insertions and/or deletions may be considered as mutations.

The publications discussed herein are provided solely for their disclosure prior to the filing date of the present application. Nothing herein is to be construed as an admission that such publications constitute prior art to the claims appended hereto.

### NUMBERED PARAGRAPHS

The present invention may be described by way of the following numbered paragraphs:
1. A modulator of CFC1 for use in treating neuroendocrine neoplasms.
2. The modulator for use according to paragraph 1, wherein the modulator:
   (i) antagonizes CFC1 activity;
   (ii) inhibits CFC1 signalling;
   (iii) inhibits the function of CFC1;
   (iv) is capable of recruiting immune cells;
   (v) is capable of targeting immune cells to a tumour or cancer, wherein the tumour or cancer expresses CFC1;
   (vi) elicits an immune response; and/or
   (vii) elicits an anti-tumoral immune response.
3. The modulator for use according to paragraph 1 or paragraph 2, wherein the modulator is an inhibitor of CFC1.
4. The modulator for use according to any of paragraphs 1 to 3, wherein the modulator is a binding molecule comprising a CFC1 binding domain.
5. The modulator for use according to paragraph 4, wherein the binding molecule comprises an anti-CFC1 antibody or a CFC1-binding antibody fragment thereof; optionally wherein the anti-CFC1 antibody or a CFC1-binding antibody fragment thereof antagonizes CFC1 activity.
6. The modulator for use according to paragraph 4 or paragraph 5, wherein the binding molecule or the CFC1-binding antibody antibody or the CFC1-binding antibody fragment thereof binds to an amino acid sequence comprising:
   (i) SEQ ID NO: 1 or a variant having at least 80% sequence identity thereto;
   (ii) SEQ ID NO: 2 or a variant having at least 80% sequence identity thereto;
   (iii) SEQ ID NO: 3 or a variant having at least 80% sequence identity thereto; or
   (iv) SEQ ID NO: 4 or a variant having at least 80% sequence identity thereto.
7. The modulator for use according to paragraph 5 or paragraph 6, wherein the anti-CFC1 antibody or CFC1-binding antibody fragment thereof comprises a heavy chain variable (VH) domain and/or a light chain variable (VL) domain.
8. The modulator for use according to paragraph 7, wherein the VH domain comprises heavy chain complementarity determining regions (HCDRs) 1-3, and/or the VL domain comprises light chain complementarity determining regions (LCDRs) 1-3, wherein (according to Kabat numbering):
   (i)
      HCDR1 comprises the amino acid sequence according to SEQ ID NO: 5 (SSIYYMC),
      HCDR2 comprises the amino acid sequence according to SEQ ID NO: 6 (CIYVGSSGSTYYASWAKG),
      HCDR3 comprises the amino acid sequence according to SEQ ID NO: 7 (GYVDYAGVTYGTDYFNL);
      and/or
   (ii)
      LCDR1 comprises the amino acid sequence according to SEQ ID NO: 8 (QASEDIYSNLA),
      LCDR2 comprises the amino acid sequence according to SEQ ID NO: 9 (GASTLAS), and
      LCDR3 comprises the amino acid sequence according to SEQ ID NO: 10 (QGGYYSRSAA);
      optionally wherein one or more of the CDRs comprises one, two or three amino acid mutations relative to the recited sequences.
9. The modulator for use according to paragraph 7, wherein the VH domain comprises heavy chain complementarity determining regions (HCDRs) 1-3, and the VL domain comprises light chain complementarity determining regions (LCDRs) 1-3, wherein (according to IMGT numbering):
   HCDR1 comprises the amino acid sequence according to SEQ ID NO: 17 (GFSFSSIYY),
   HCDR2 comprises the amino acid sequence according to SEQ ID NO: 18 (IYVGSSGST),
   HCDR3 comprises the amino acid sequence according to SEQ ID NO: 19 (ARGYVDYAGVTYGTDYFNL),
   LCDR1 comprises the amino acid sequence according to SEQ ID NO: 20 (EDIYSN), LCDR2 comprises the amino acid sequence GAS, and
   LCDR3 comprises the amino acid sequence according to SEQ ID NO: 21 (QGGYYSRSAA);
   optionally wherein one or more of the CDRs comprises one, two or three amino acid mutations relative to the recited sequences.
10. The modulator for use according to paragraph 7, wherein the VH domain comprises heavy chain complementarity determining regions (HCDRs) 1-3, and/or the VL domain comprises light chain complementarity determining regions (LCDRs) 1-3, wherein (according to Kabat numbering):
   (i)
      HCDR1 comprises the amino acid sequence according to SEQ ID NO: 11 (SSYGVS),
      HCDR2 comprises the amino acid sequence according to SEQ ID NO: 12 (YIDPVFDSTYYASWVNG),
      HCDR3 comprises the amino acid sequence according to SEQ ID NO: 13 (GGMVIRL);
         and/or
   (ii)
      LCDR1 comprises the amino acid sequence according to SEQ ID NO: 14 (QASQSISTALA),
      LCDR2 comprises the amino acid sequence according to SEQ ID NO: 15 (DASKLAS), and
      LCDR3 comprises the amino acid sequence according to SEQ ID NO: 16 (QHGYYSSSTVYNA);
      optionally wherein one or more of the CDRs comprises one, two or three amino acid mutations relative to the recited sequences.
11. The modulator for use according to paragraph 7, wherein the VH domain comprises heavy chain complementarity determining regions (HCDRs) 1-3, and the VL domain comprises light chain complementarity determining regions (LCDRs) 1-3, wherein (according to IMGT numbering):
   HCDR1 comprises the amino acid sequence according to SEQ ID NO: 22 (GFDFSSYG),
   HCDR2 comprises the amino acid sequence according to SEQ ID NO: 23 (IDPVFDST),
   HCDR3 comprises the amino acid sequence according to SEQ ID NO: 24 (VQGGMVIRL),
   LCDR1 comprises the amino acid sequence according to SEQ ID NO: 25 (QSISTA), LCDR2 comprises the amino acid sequence DAS, and
   LCDR3 comprises the amino acid sequence according to SEQ ID NO: 26 (QHGYYSSSTVYNA);
   optionally wherein one or more of the CDRs comprises one, two or three amino acid mutations relative to the recited sequences.
12. The modulator for use according to paragraph 8 or paragraph 9, wherein:
   (i) the VH domain comprises a sequence having at least 70%, at least 75%, at least 80%, at least 85%, at least 90%, at least 95%, at least 97%, at least 99%, or 100% identity to SEQ ID NO: 27; and/or
   (ii) the VL domain comprises a sequence having at least 70%, at least 75%, at least 80%, at least 85%, at least 90%, at least 95%, at least 97%, at least 99%, or 100% identity to SEQ ID NO: 28.
13. The modulator for use according to paragraph 10 or paragraph 11, wherein:
   (i) the VH domain comprises a sequence having at least 70%, at least 75%, at least 80%, at least 85%, at least 90%, at least 95%, at least 97%, at least 99%, or 100% identity to SEQ ID NO: 29; and/or
   (ii) the VL domain comprises a sequence having at least 70%, at least 75%, at least 80%, at least 85%, at least 90%, at least 95%, at least 97%, at least 99%, or 100% identity to SEQ ID NO: 30.
14. The modulator for use according to any of paragraphs 7 to 13, wherein the CFC1-binding antibody or CFC1-binding antibody fragment thereof comprises:
   (i) a constant heavy chain comprising a sequence having at least 70%, at least 75%, at least 80%, at least 85%, at least 90%, at least 95%, at least 97%, at least 99%, or 100% identity to SEQ ID NO: 31; and/or
   (ii) a constant light chain comprising a sequence having at least 70%, at least 75%, at least 80%, at least 85%, at least 90%, at least 95%, at least 97%, at least 99%, or 100% identity to SEQ ID NO: 32.
15. The modulator for use according to any of paragraphs 7 to 14, wherein the CFC1-binding antibody or CFC1-binding antibody fragment thereof comprises:
   (i) a VH domain comprising a sequence having at least 70%, at least 75%, at least 80%, at least 85%, at least 90%, at least 95%, at least 97%, at least 99%, or 100% identity to SEQ ID NO: 27;
   (ii) a VL domain comprising a sequence having at least 70%, at least 75%, at least 80%, at least 85%, at least 90%, at least 95%, at least 97%, at least 99%, or 100% identity to SEQ ID NO: 28;
   (iii) a constant heavy chain comprising a sequence having at least 70%, at least 75%, at least 80%, at least 85%, at least 90%, at least 95%, at least 97%, at least 99%, or 100% identity to SEQ ID NO: 31; and
   (iv) a constant light chain comprising a sequence having at least 70%, at least 75%, at least 80%, at least 85%, at least 90%, at least 95%, at least 97%, at least 99%, or 100% identity to SEQ ID NO: 32.
16. The modulator for use according to paragraph 15, wherein the CFC1-binding antibody or CFC1-binding antibody fragment thereof comprises:
   (i) a VH domain comprising a sequence as set forth in SEQ ID NO: 27;
   (ii) a VL domain comprising a sequence as set forth in SEQ ID NO: 28;
   (iii) a constant heavy chain comprising a sequence as set forth in SEQ ID NO: 31; and
   (iv) a constant light chain comprising a sequence as set forth in SEQ ID NO: 32.
17. The modulator for use according to any of paragraphs 7 to 14, wherein the CFC1-binding antibody or CFC1-binding antibody fragment thereof comprises:
   (i) a VH domain comprising a sequence having at least 70%, at least 75%, at least 80%, at least 85%, at least 90%, at least 95%, at least 97%, at least 99%, or 100% identity to SEQ ID NO: 29;
   (ii) a VL domain comprising a sequence having at least 70%, at least 75%, at least 80%, at least 85%, at least 90%, at least 95%, at least 97%, at least 99%, or 100% identity to SEQ ID NO: 30;
   (iii) a constant heavy chain comprising a sequence having at least 70%, at least 75%, at least 80%, at least 85%, at least 90%, at least 95%, at least 97%, at least 99%, or 100% identity to SEQ ID NO: 31; and
   (iv) a constant light chain comprising a sequence having at least 70%, at least 75%, at least 80%, at least 85%, at least 90%, at least 95%, at least 97%, at least 99%, or 100% identity to SEQ ID NO: 32.
18. The modulator for use according to according to paragraph 17, wherein the CFC1-binding antibody or CFC1-binding antibody fragment thereof comprises:
   (i) a VH domain comprising a sequence as set forth in SEQ ID NO: 29;
   (ii) a VL domain comprising a sequence as set forth in SEQ ID NO: 30;
   (iii) a constant heavy chain comprising a sequence as set forth in SEQ ID NO: 31; and
   (iv) a constant light chain comprising a sequence as set forth in SEQ ID NO: 32.
19. The modulator for use according to any of paragraphs 5 to 7, wherein the CFC1-binding antibody is antibody clone MM0205-8D12
20. The modulator for use according to any of paragraphs 5 to 7, wherein the CFC1-binding antibody is antibody M09 clone 2G4.
21. A polynucleotide for use in treating neuroendocrine neoplasms, wherein the polynucleotide comprises a nucleic acid sequence encoding the modulator as defined in any of paragraphs 1-20.
22. The polynucleotide for use according to paragraph 21, wherein the nucleic acid sequence is an RNA sequence.
23. A vector for use in treating neuroendocrine neoplasms, wherein the vector comprises the polynucleotide as defined in paragraph 21 or paragraph 22.
24. A composition for use in treating neuroendocrine neoplasms, wherein the composition comprises the modulator of any of paragraphs 1 to 20, or the polynucleotide as defined in paragraph 21 or paragraph 22, or the vector as defined in paragraph 23.
25. A pharmaceutical composition for use in treating neuroendocrine neoplasms, wherein the pharmaceutical composition comprises the modulator of any of paragraphs 1 to 20, the polynucleotide as defined in paragraph 21 or paragraph 22, or the vector as defined in paragraph 23, or the composition for use as defined in paragraph 24; together with a pharmaceutically acceptable carrier, diluent or excipient.
26. The pharmaceutical composition for use according to paragraph 25, further comprising a vesicle, a nanoparticle, a lipid nanoparticle (LNP), a liposome or a polymeric mixture.
27. The pharmaceutical composition for use according to paragraph 26, wherein the modulator, the polynucleotide, the vector, or the composition is encapsulated within the vesicle, nanoparticle, LNP, liposome or polymeric mixture.
28. The modulator for use according to any of paragraphs 1 to 20, the polynucleotide for use according to paragraph 21 or paragraph 22, the vector for use according to paragraph 23, the composition for use according to paragraph 24, or the pharmaceutical composition for use according to any of paragraphs 25 to 27; wherein the neuroendocrine neoplasm is a tumour or cancer expressing CFC1, wherein the expression of CFC1 is increased compared to the expression of CFC1 by a non-cancerous tissue.
29. The modulator for use according to any of paragraphs 1 to 20, the polynucleotide for use according to paragraph 21 or paragraph 22, the vector for use according to paragraph 23, the composition for use according to paragraph 24, or the pharmaceutical composition for use according to any of paragraphs 25 to 27, and/or the modulator for use, the polynucleotide for use, the vector for use, the composition for use, or the pharmaceutical composition for use according to paragraph 28, wherein the neuroendocrine neoplasm is selected from the list consisting of: colorectal a neuroendocrine tumor, a pancreatic neuroendocrine tumor, a gastroenteropancreatic (GEP) tumour, and a pulmonal neuroendocrine tumor.
30. The modulator for use according to any of paragraphs 1 to 20, the polynucleotide for use according to paragraph 21 or paragraph 22, the vector for use according to paragraph 23, the composition for use according to paragraph 24, or the pharmaceutical composition for use according to any of paragraphs 25 to 27, and/or the modulator for use, the polynucleotide for use, the vector for use, the composition for use, or the pharmaceutical composition for use according to paragraph 28, wherein the neuroendocrine neoplasm is not neuroblastoma.

The invention will now be further described by way of Examples, which are meant to serve to assist one of ordinary skill in the art in carrying out the invention and are not intended in any way to limit the scope of the invention.

### EXAMPLES

### Example 1 - Expression Analysis of CFC1 in Tumor and Normal Tissue using RNA-Seq Data

RNA sequencing data of neuroendocrine neoplasms (NEN) and healthy tissues was explored in order to select for the most frequently and tumor-specifically expressed target genes. Targets were evaluated for a diverse cohort of neuroendocrine neoplasms (major subtypes are rectal, small intestinal and pancreatic NENs).

CFC1 was identified as being abundantly expressed in neuroendocrine neoplasms, but not or only in a restricted manner in normal tissues.

All in silico analyses were done using publicly available normal tissue expression data from the GTEx, Genotype-Tissue Expression project (Nature genetics 45, 580-585 (2013)) and gastroenteropancreatic (GEP) NEN expression data (Nature Genetics 50, 979-989 (2018)) and proprietary RNA-Seq gene expression data.

Public and in-house generated RNA-Seq gene expression data is derived from 3809 normal tissue samples representing 37 tissue sites and 310 GEP-NEN samples consisting of 147 metastasis samples and 163 primary samples. Major GEP-NEN tissue sites include 58 rectal GEP-NEN samples, 49 small intestinal GEP-NEN samples and 115 pancreatic GEP-NEN samples.

All analyzed samples were derived from fresh-frozen (FF) sample material. For in-house samples total RNA was extracted using Qiagen's RNeasy tissue mini kit. Sequencing libraries were generated using Illumina's TruSeq stranded mRNA kit: mRNA molecules containing poly-A were purified using magnetic beads bound to poly-T-oligo. After purification, the mRNA was fragmented into small pieces. RNA fragments were copied into first strand cDNA using reverse transcriptase and random primers, followed by second strand cDNA synthesis using DNA Polymerase I and RNase H. These cDNA fragments then had the addition of a single 'A' base and subsequent ligation of the adapter. The products were then purified and PCR-enriched to create the final cDNA library. Sequencing libraries were further checked for quantity and quality using Qubit fluorometer and Agilent Bioanalyzer 2100. The libraries were sequenced 2-plex in paired-end mode (2 x 50 nt) on a HiSeq2500 HS V3 flow cell resulting in ~150-200 million distinct sequencing reads per library.

RNA sequencing reads were aligned to the hg19 reference genome and transcriptome, and gene expression was determined by comparison with UCSC known genes transcript and exon coordinates, followed by normalization to Reads Per Kilobase per Million mapped reads (RPKM) units (Mortazavi, A. et al., Nature methods 5, 621-628 (2008); Langmead, B. et al., Genome biology 10, R25; (2009)). Targets were selected by comparing expression in tumor and normal tissues, and to achieve a high coverage across the tumor cohorts. Target-expressing tumors were defined by expression value > 1 RPKM.

Based on RNA-Seq data, CFC1 is expressed in almost all analysed neuroendocrine neoplasm (NEN) samples (95 % >1 RPKM, Figure 1). Most NEN samples show very high CFC1 expression values (median expression around 1000 RPKM). Expression was observed in 100 % of primary pancreatic NENs and in 83 % of pancreatic NEN metastasis, in 95 % of primary small intestinal NEN and in 100% of small intestinal NEN metastasis and in 87 % of primary rectal NENs and in 97 % of rectal NEN metastasis (>1 RPKM, Figure 1). Furthermore, normal tissues were analyzed to ensure a tumor-specific expression of CFC1 and to exclude high expression of CFC1 in normal tissues like brain, heart, liver, kidney and lung associated with higher potential risk of toxicity in clinical trials (Figure 1).

### Example 2 - CFC1 RNA and protein expression in tumor samples and tumor adjacent tissues

For immunohistochemistry (IHC), staining conditions for commercially available antibodies were established in order to confirm expression of CFC1 in human FFPE (formaldehyde-fixed paraffine-embedded) tissue samples. FFPE samples were first sliced into 3 µm sections, followed by dewaxing in the stainer Stainmate Max. Antigen retrieval was then performed by incubating sections in Citrate buffer for 30 minutes at 95 °C. Afterwards, sections were quenched for 15 minutes in 0.3% H₂O₂ solution, and blocked for 30 minutes in blocking solution (10% goat serum in PBS). The CFC1 antigen was detected by incubation with the purified polyclonal primary antibody (1:2000) overnight at 4 °C (Table 2).

**Table 21: Antibodies used for IHC for CFC1**

| **Target gene** | **Antibody Manufacture** | **Clonality** |
|---|---|---|
| CFC1 | Invitrogen, Waltham, MA, USA | Polyclonal |

Next day, samples were incubated with HPR-conjugated secondary antibody anti-rabbit for 30 minutes at RT, followed by nuclear staining with Hoechst (1:5000 in PBS) for 3 minutes. The staining was detected using a streptavidin-HRP system. Counterstaining was performed with hematoxylin for 1 minute. For dehydration, sections were immersed in Ethanol, following by 2 times 5 minutes incubation in Xylol. Finally, samples were mounted with aqueous mounting medium Immumount Shandon. Slides were imaged with the Aperio AT2 Scanner and analysed with ImageScope software.

IHC positive samples for CFC1 include samples across all analysed entities. Figure 2 shows a correlation with CFC1 RNA expression by qRT-PCR. Here results between qRT-PCR and IHC are highly correlated.

### Example 3 - Subcellular localization of CFC1

To assess the subcellular localization of CFC1, immunofluorescent staining was performed on NCI-H810 cells that endogenously express CFC1 (Figure 3). For this, coverslips were coated with collagen for 30 minutes. Next, cells were harvested and transferred to the coated coverslips. Adherent cells were fixed in 2 % paraformaldehyde for 20 min at RT, permeabilized for 5 min at RT in PBST++ (PBS++ mixed with 0.1 % Triton). Subsequently, samples were quenched for 5 min at RT in PBS++ containing 50 mM NH₄Cl and blocked for 1 hour at RT in PBST++ containing 1 % BSA. Cells were then incubated with a primary antibody against CFC1 for 1 hour at RT, followed by an incubation with a fluorescently labelled secondary antibody along with phalloidin (stains cytoskeleton), concanavalin (stains endoplasmic reticulum), lectin HPA (stains golgi apparatus) as well as Hoechst (nuclei) (Table 3). Cells were mounted with mounting media and visualized using the Leica SP8 confocal microscope.

**Table 32: Stains used for IF.**

| **Stains** | **Antibody Manufacture** |
|---|---|
| **Concanavalin A** | Invitrogen, Waltham, MA, USA |
| **Lectin HPA** | Invitrogen, Waltham, MA, USA |
| **Hoechst** | Invitrogen, Waltham, MA, USA |

To determine cell surface expression of CFC1, a biotinylation assay was performed using NCI-H810 cells stably over-expressing CFC1 (Figure 3B). For this, cells were plated in two 10-cm dishes and grown to 80% confluence. Afterwards, cells were incubated with either 0.5 mg/mL EZ-Link-Sulfo-NHS-Biotin or PBS (nonbiotinylated control) for 2 h on ice. The reaction was quenched by incubation with 50 mM glycine/PBS solution for 10 min. The cells were transferred to a reaction tube and sonified. The lysates were incubated for 30 min at 4°C followed by a centrifugation. Streptavidin-agarose (SA) beads were washed with RIPA buffer and split into 2 reaction tubes. Supernatants were removed and the lysates were added except for the input. Lysates were incubated for 4 h at 4°C on a rotator. The Protein-Biotin-SA bead complexes were pelleted by centrifugation and the supernatants were removed (flow through). The pellets were washed three times with RIPA buffer and one time with 0.9% NaCl, prior to resuspension in 4x sample buffer and boiling for 10 min at 98°C and 400 rpm. After a short centrifugation, eluates were taken off. The remaining beads were resuspended in 4x SDS sample buffer. Lysates, input, eluates and flow through were separated by SDS-PAGE using a 10% gel matrix. Protein transfer on nitrocellulose membrane was performed by semi-dry blot. Immunostainings with antibodies raised against the plasma membrane markers Na/K ATPase and Caveolin (CAV-1) were used as positive control. Antibodies raised against intracellular proteins such as MEK1/2, p84 and alpha-Tubulin were used as negative control (Figure 3B). Since only cell surface proteins such as Na/K ATPase and CAV-1 were detectable in the biotinylated pull-down fraction (Eluat) but not MEK1/2, p84 and alpha-Tubulin, isolation of the plasma membrane was successful. The Flowthrough, which should contain remaining intracellular proteins revealed an expression of MEK1/2, alpha tubulin and p84 as expected. CAV-1 showed up in the eluate as well as in the flow through suggesting that Caveolin is present in both, plasma membrane and cytoplasm. CFC1 protein was detectable in the biotinylated fraction indicating that CFC1 is expressed on the cell surface and can be targeted from the extracellular site (Figure 3B).

**Table 43: Antibodies used for streptavidin Western blot analysis.**

| **Target gene** | **Antibody Manufacture** | **Clonality** | **Clone number** |
|---|---|---|---|
| CFC1 | R&D Systems, Minneapolis, MN, US | Monoclonal | 219708 |
| **Na+/K+ ATPase** | GeneTex, Irvine, CA, USA | Monoclonal | EP1845Y |
| **Caveolin-1** | ThermoFisher Scientific, Waltham, MA, USA | Polyclonal | |
| **MEK1/2** | Cell Signaling, Danvers, MA, USA | Monoclonal | L38C12 |
| **p84** | GeneTex, Irvine, CA, USA | Monoclonal | 5E10 |
| **α-tubulin** | Sigma-Aldrich, St. Louis, MO, USA | Monoclonal | B-5-1-2 |

Furthermore, cell surface expression of CFC1 was assessed by multiplex immunofluorescent staining of tissue sections from a patient with rectal NEN. For multiplex immunohistochemical stains, 3.0 µm thick sections of FFPE tissue were generated using a rotary microtome. Dewaxing took place in the stainer Stainmate Max. Epitope retrieval was performed with heat-induced epitope retrieval in 10 mM citric buffer pH 6 with 0.05% Tween20 for 10 min at 120°C with Pascal pressure cooker. Endogenous peroxidases were subsequently quenched using 0.3% H₂O₂ solution in PBS for 15 min, followed by incubation with 10% goat serum in PBS for 30 min to block the unspecific antibody binding sites. The CFC1 antigen was detected by incubation with the purified polyclonal primary antibody at RT for 1h followed by the incubation with the BrightVision polymer HRP-conjugated anti-rabbit secondary antibody at room temperature (RT) for 30min. Signal is amplified by Opal 570 dye (tyramide is labelled with Opal 570) for 10 min at RT. First primary antibody-secondary antibody complex was then stripped with heat-induced epitope retrieval in 10 mM citric buffer pH 6 with 0.05% Tween20 for 10 min at 120°C with Pascal pressure cooker. After repetition of blocking step, CD56 was detected by incubation with the purified monoclonal primary anti-CD56 antibody overnight at 2°C to 8°C followed by the incubation with the BrightVision polymer HRP-conjugated anti-rabbit secondary antibody at room temperature (RT) for 30 min. Signal is amplified by Opal 520 dye (tyramide is labelled with Opal 570,) for 10 min at RT. Nuclear counterstaining was performed with incubating the slides in DAPI for 5 min at RT followed by mounting with aqueous mounting medium Immumount Shandon.Analysis and documentation were performed using the AxiolmagerM2 (Zeiss).

**Table 54: Antibodies used for IF.**

| **Target gene** | **Antibody Manufacture** | **Clonality** | **Clone number** |
|---|---|---|---|
| **CFC1** | Invitrogen, Waltham, MA, USA | Polyclonal | |
| **CD56** | Cell Marque, Rocklin, CA, USA | Monoclonal | MRQ-42 |

Immunofluorescent staining of rectal NEN revealed that CFC1 is only co-expressed with CD56, indicating its localization at the membrane of CD56-positive NEN cells. CD56 (also known as neural cell adhesion molecule, NCAM) is a cell surface protein and commonly used as a marker for neuroendocrine neoplasms (Figure 3C).

### Example 4 - Biological function of CFC1 in neuroendocrine neoplasm cell lines

CFC1 was further characterised by analysis of neuroendocrine neoplasm cell lines NCI-H810 and NCI-H727. NCI-H810 and NCI-H727 show moderate endogenous expression of CFC1, as determined by flow cytometry staining. NCI-H810 cell line was sorted into a CFC1 high expressing cell line and CFC1 low expressing cells. Furthermore, for NCI-H810 and NCI-H727 cell lines, CFC1 overexpressing cell lines were generated as well.

To assess the molecular function of CFC1, the established cell lines were used to determine cell proliferation, tumor sphere and colony formation, as well as migration and invasion.

To determine cell proliferation, the Click-iT^{®} EdU Flow Cytometry Assay Kit was used. EdU was added to the culture medium and incubated for 1 to 2 hours. Cells were detached with Accutase, followed by extracellular staining of CFC1. For this, cells were incubated with anti-CFC1 mAb for 30 minutes at 4 °C. Next, cells were incubated with a secondary antibody for 20 minutes at 4 °C, and subjected to intranuclear staining of EdU according to manufacturer's instructions (Figure 4A).

For tumor sphere formation assays, cells were first detached with Accutase, and the cell suspension was centrifuged at 300xg for 4 minutes at room temperature. Pelleted cells were resuspended and strained with a 70 µm cell strainer. Then, cells were counted and 100 to 200 cells were plated in each well of an ultra-low attachment 96-well flat bottom plate. After 21 days, tumor spheres were counted using a transmitted light microscope with a 10x objective (Figure 4B).

For colony formation assays, cells were harvested with Accutase and centrifuged at 300xg for 4 minutes. Cell pellets were resuspended in 10 ml medium and filtered through a 70 µm cell strainer. After cell counting, 60 to 100 cells were directly seeded into a 24-well. Two weeks later, medium was removed and each well was washed once with 1x PBS. Subsequently, one ml of fixation-staining solution was added to each well and incubated for 30 min. After staining solution was removed, wells were washed carefully with VE water. Plates were air dried and colonies were counted (Figure 4C).

A 3D invasion assay was used to evaluate the impact of NE cell lines on cell invasion. NCI-H727 cells were cultivated to form spheroids. After 10 days of cultivation, cold matrigel mixture was added to spheroids (10% matrigel in serum free media). Spheroids were imaged on day 0 and day 10 under the microscope (Figure 4D).

Migration of NCI-H810 cell lines was determined by performing a Migration and Invasion Assay (MANIA). NCI-H810 cells were plated on 10 cm dish. After 48h, cells were stained with Cell Tracker Orange in serum free medium for 30 min at 37 °C. Medium was removed and cells were detached with Accutase. After centrifugation, pellets were resuspended in serum free media and cells were plated in 96 plate well. After 24 hours, cells were washed with starvation media and cold matrigel mixture (10% matrigel in serum free media) was added to cells. To harden the matrigel, plates were placed in the incubator for 30 min, following adding 50 µl of full media dropwise to the matrigel mixture. Migration and invasion of cells was imaged on day 2 at the Leica SP8 confocal microscope (Figure 4E).

Differences in sphere number were observed as well as in colony number. CFC1 high expressing and CFC1 overexpressing cell lines overall generated more colonies and more tumor spheres compared to cells with lower expression of CFC1. Moreover, cells with elevated expression of CFC1 revealed a higher potential for migration and invasion but not proliferation.

### Example 5 - CFC1-dependent signalling in neuroendocrine neoplasm cell lines

To determine signalling pathways involved in promoting colony formation downstream of CFC1, inhibitors of certain signalling molecules were used. NEN colonies and spheroids were plated as described above. After plating of cells, different concentration of inhibitors against ALK4/5/7 (A83), IGFR1R (AG1024), SMAD3 (SIS3), AKT (MK2206), and ERK (SCH7298) were added to the medium (Figure 5B-D). Successful inhibition of CFC1-dependent signalling would result in diminished colony formation compared to untreated colonies. CFC1-dependent colony formation was evaluated after 14 days (Figure 5B and 5C) and sphere formation was assessed after 21 days (Figure 5D).

### Example 6 - CFC1-dependent signalling in NEN tissue

To assess whether CFC1/SMAD signalling is upregulated in human GEP-NENs, single and multiplex immunohistochemistry (IHC) staining were performed. FFPE samples were first sliced into 3 µm sections, followed by dewaxing in the stainer Stainmate Max. Antigen retrieval was then performed by incubating sections in Citrate buffer for 30 minutes at 95 °C. Afterwards, sections were quenched for 15 minutes in 0.3% H₂O₂ solution, and blocked for 30 minutes in blocking solution (10% goat serum in PBS). Staining with primary antibody (Table 6) was performed overnight at 4 °C. The next day, samples were incubated with HRP-conjugated secondary antibody for 30 minutes at RT. In case of single staining, samples were stained with Hoechst for 3 minutes to stain the nuclei. The staining was detected using a streptavidin-HRP system. Counterstaining was performed with hematoxylin for 1 minute. For dehydration, sections were immersed in Ethanol, following by 2 times 5 minutes incubation in Xylol. Finally, samples were mounted with aqueous mounting medium Immumount Shandon. Slides were imaged with the Aperio AT2 Scanner and analysed with ImageScope software. In the case of multiplex IHC, after incubation with secondary antibody, samples were amplified with Opal fluorophore (Opal 480, Opal 520, Opal 570 and Opal 690) for 10 minutes at RT. To stain samples with the next antibody, they were stripped in citrate buffer for 20 minutes at 95 °C and went through the same steps from blocking to amplification. Lastly, final antibody stripping was performed, following nuclear staining with Spectral DAPI, and mounting. Imaging was performed with Vectra Polaris multispectral slide scanner and analysis with Phenochart software (Figure 6A-D).

**Table 65: Antibodies used for IHC/IF.**

| **Target gene** | **Antibody Manufacture** | **Clonality** | **Clone number** |
|---|---|---|---|
| **CFC1** | Invitrogen, Waltham, MA, USA | Polyclonal | |
| **Nodal** | Abcam, Cambridge, UK | Polyclonal | |
| **SMAD2/3** | Sigma-Aldrich, St. Louis, MO, USA | Monoclonal | D7G7 |
| **pSMAD3** | Abcam, Cambridge, UK | Monoclonal | ST0493 |

RNA-Seq was conducted as described in section 1. From obtained data, differential gene expression analysis was performed using DESeq2 (version v1.34.0) (PMID: 25516281) to identify genes with significant expression differences between sample groups (Figure 6E).

### Example 7 - Tumorigenic effect of CFC1 in vivo

To evaluate a pro-tumorigenic function of CFC1, NCI-H810 cells sorted for high and low CFC1 expression were injected subcutaneously into immunocompromised mice. Tumor cells were detached with Accutase and resuspended in DPBS. The cells were kept on ice until injection and mixed with Matrigel in a 1:1 ratio. Mice were first anaesthetized with 2.5 % isoflurane in 0₂. Subsequently, 5 x 10⁶ cells/100 µl cells was injected subcutaneously into the flanks of mice using a 0.5 mL insulin syringe. Tumor growth was measured unblinded every 2-4 days using a calliper, and volumes were calculated with the formula $V = \frac{{A}^{2} \times B}{2}$ (A = width, B = length). Mice were sacrificed by cervical dislocation upon reaching endpoint criteria, with daily health assessments and increased monitoring if symptoms worsened.

### Housing Condition and Husbandry

Mice were housed under specific pathogen-free (SPF) conditions in accordance with federal and state policies on animal research. The animal facility was ventilated with 8-10 room air changes per hour, with humidity adjusted to 45-66 % and temperatures between 21 °C and 24 °C. For the experiments, NOD.Cg-Prkdc scid II2rg tm1Wjl /SzJ (bred in-house) mice strains were used. Female animals, age-matched at 6 to 8 weeks, were used throughout all experiments. Experiments were approved by the regulatory authorities for animal welfare.

### Preparation of tumor cells

Tumors were prepared by excising their base and removing the overlying skin along with the adjacent normal tissue using fine scissors. The tumors were then weighed and placed into reaction tubes containing 10 mL of DPBS. To generate single-cell suspensions (SCS), tumors were transferred to C tubes, cut into smaller pieces, and digested in digestion buffer using the mouse tumor dissociation and gentleMACS^{™} Octo Dissociator. Samples were then incubated at 37 °C for 40 minutes and passed through a 70 µm cell strainer, washed with RPMI 1640, and centrifuged at 460 x g for 6 minutes. The resulting cell pellets were resuspended in hypotonic electrolyte solution to induce erythrocyte lysis. The suspension was incubated at room temperature for 3 minutes, and the lysis reaction was stopped by adding DPBS/2 mM EDTA. The cell suspension was then filtered through a 70 µm strainer and centrifuged at 460 x g for 6 minutes. Finally, the cell pellets were resuspended in DPBS.

### Sample Collection and Processing

Tumors were removed after euthanization and tissue pieces were split for IHC and flow cytometry. Tumor tissue for IHC analysis were stored in Histofix immediately after tumor excision. Staining for CFC1 was performed as described in section 1.4. Single cell suspensions for flow cytometry were prepared as follows: The removed organs are pressed through a 70 µm cell mesh using the plunger of a syringe to release the cells from the organ into a tube. After washing with PBS the cell pellet is incubated with erythrocyte lysis buffer, washed in PBS and passed again through a 70 µm cell mesh. In the end the cells are resuspended in medium and counted. For flow cytometry a AF488 conjugated CFC1 antibody (ID:464) was used to determine CFC1 expression in the different tumor samples (Figure 7).

### Example 8 - Treatment with CFC1-specific antibodies

Antibodies were tested for their capability to reduce CFC1 related SMAD2 and SMAD3 phosphorylation after induction with Nodal (Figure 8A and 8B; Figure 9C). For this, NCI-H810 cells overexpressing CFC1 were pre-incubated with antibodies for 30 min, followed by stimulation with 100 ng/mL Nodal for one hour. Subsequently, cells were lysed, and total protein concentrations were determined using BCA-Assay. For SDS-page, total cell lysates were loaded onto SDS-polyacrylamide gels and subjected to Western blot, followed by semi-dry transfer onto a nitrocellulose membrane. Immunoblotting was performed using antibodies specific for phospho-SMAD2, phospho-SMAD3, SMAD2/3, GAPDH, and α-Tubulin (Table 7). Western blot signals were detected using Image Quant LAS4000, and the phosphorylated SMAD2/3-to-total SMAD2/3 protein ratio was quantified by densitometric analysis (Figure 8A and 8B as well as Figure 9C).

We identified two CFC1-specific antibodies ID:464 and ID:638 that block SMAD2 and SMAD3 phosphorylation very efficiently **(Error! Reference source not found.8A,** B).

**Table 76: Antibodies used for Western blot.**

| **Target gene** | **Antibody Manufacture** | **Clonality** | **Clone number** |
|---|---|---|---|
| **SMAD3** | Cell Signaling, Danvers, MA, USA | Monoclonal | D7G7 |
| **pSMAD2** | Cell Signaling, Danvers, MA, USA | Monoclonal | E8F3R |
| **pSMAD3** | Abcam, Cambridge, UK | Monoclonal | EP823Y |
| **GAPDH** | GeneTex, Irvine, CA, USA | Monoclonal | GT239 |
| **α-Tubulin** | Sigma-Aldrich, St. Louis, MO, USA | Monoclonal | B-5-1-2 |

Binding of CFC1-specific monoclonal antibodies ID:637 and ID:638 was determined with flow cytometry. For surface protein analysis, cells were incubated with CFC1-specific antibodies for 30 minutes at 4 °C. Cells were then incubated with a secondary antibody for 20 minutes at 4 °C, and fixated with BD^{™} Stabilizing Fixative for 15 minutes at 4 °C. Flow cytometry was conducted using FACSCanto^{™} II. Data were analyzed using FlowJo 10 software (Figure 8C).

**Table 87: CFC1 specific antibodies.**

| **Internal number** | **Antibody Manufacture** | **Clonality** | **Clone number** |
|---|---|---|---|
| **ID:464** | R&D Systems | Monoclonal | 219708 |
| **ID:534** | Abcam | Polyclonal | |
| **ID:535** | ThermoFisher Scientific | Polyclonal | |
| **ID:536** | Sino Biological/Biozol | Polyclonal | |
| **ID:637** | Novus Biologicals | Monoclonal | 2G4 |
| **ID:638** | Novus Biologicals | Monoclonal | MM0205-8D12 |
| **ID:462** | Sigma Aldrich | Monoclonal | 2G4 |
| **ID:463** | | Polyclonal | |
| **Clone 1** | BioNTech | Monoclonal | |
| **Clone 2** | BioNTech | Monoclonal | |

To determine whether specific binding of antibodies can suppress CFC1-dependent colony formation, we used CFC1-specific monoclonal antibodies ID:638 as efficient CFC1 binder (as seen in Figures 8A-C) and ID:637 as negative control (Figure 8D), as well as clone 1 and clone 2 (Figure 9B). NET colonies were plated as described above and treated with 1 µg/ml of CFC1-specific monoclonal antibodies 24 hours after seeding. CFC1-dependent colony formation was evaluated after 14 days (Figure 8D and Figure 9B).

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A modulator of CFC1 for use in treating neuroendocrine neoplasms

2. The modulator for use according to claim 1, wherein the modulator:
(i) antagonizes CFC1 activity;
(ii) inhibits CFC1 signalling;
(iii) inhibits the function of CFC1;
(iv) is capable of recruiting immune cells; and/or
(v) is capable of targeting immune cells to a tumour or cancer, wherein the tumour or cancer expresses CFC1;
(vi) elicits an immune response; and/or
(vii) elicits an anti-tumoral immune response.

3. The modulator for use according to claim 1 or claim 2, wherein the modulator is an inhibitor of CFC1.

4. The modulator for use according to any of claims 1 to 3, wherein the modulator is a binding molecule comprising a CFC1 binding domain; optionally wherein the binding molecule comprises an anti-CFC1 antibody or a CFC1-binding antibody fragment thereof, preferably wherein the anti-CFC1 antibody or a CFC1-binding antibody fragment thereof antagonizes CFC1 activity

5. The modulator for use according to claim 4, wherein the:
(i) the anti-CFC1 antibody or the anti-CFC1 antibody fragment thereof comprises a heavy chain variable (VH) domain and/or a light chain variable (VL) domain; wherein the VH domain comprises heavy chain complementarity determining regions (HCDRs) 1-3, and/or the VL domain comprises light chain complementarity determining regions (LCDRs) 1-3, wherein (according to Kabat numbering):
(a)
HCDR1 comprises the amino acid sequence according to SEQ ID NO: 5 (SSIYYMC),
HCDR2 comprises the amino acid sequence according to SEQ ID NO: 6 (CIYVGSSGSTYYASWAKG),
HCDR3 comprises the amino acid sequence according to SEQ ID NO: 7 (GYVDYAGVTYGTDYFNL);
and/or
(b)
LCDR1 comprises the amino acid sequence according to SEQ ID NO: 8 (QASEDIYSNLA),
LCDR2 comprises the amino acid sequence according to SEQ ID NO: 9 (GASTLAS), and
LCDR3 comprises the amino acid sequence according to SEQ ID NO: 10 (QGGYYSRSAA);
optionally wherein one or more of the CDRs comprises one, two or three amino acid mutations relative to the recited sequences;
or
(ii) the anti-CFC1 antibody or the anti-CFC1 antibody fragment thereof comprises a heavy chain variable (VH) domain and/or a light chain variable (VL) domain; wherein the VH domain comprises heavy chain complementarity determining regions (HCDRs) 1-3, and/or the VL domain comprises light chain complementarity determining regions (LCDRs) 1-3, wherein (according to Kabat numbering):
(a)
HCDR1 comprises the amino acid sequence according to SEQ ID NO: 11 (SSYGVS),
HCDR2 comprises the amino acid sequence according to SEQ ID NO: 12 (YIDPVFDSTYYASWVNG),
HCDR3 comprises the amino acid sequence according to SEQ ID NO: 13 (GGMVIRL);
and/or
(b)
LCDR1 comprises the amino acid sequence according to SEQ ID NO: 14 (QASQSISTALA),
LCDR2 comprises the amino acid sequence according to SEQ ID NO: 15 (DASKLAS), and
LCDR3 comprises the amino acid sequence according to SEQ ID NO: 16 (QHGYYSSSTVYNA);
optionally wherein one or more of the CDRs comprises one, two or three amino acid mutations relative to the recited sequences; or

6. The modulator for use according to claim 5, wherein
(i)
(a) the VH domain comprises a sequence having at least 70%, at least 75%, at least 80%, at least 85%, at least 90%, at least 95%, at least 97%, at least 99%, or 100% identity to SEQ ID NO: 27; and/or
(b) the VL domain comprises a sequence having at least 70%, at least 75%, at least 80%, at least 85%, at least 90%, at least 95%, at least 97%, at least 99%, or 100% identity to SEQ ID NO: 28;
or
(ii)
(a) the VH domain comprises a sequence having at least 70%, at least 75%, at least 80%, at least 85%, at least 90%, at least 95%, at least 97%, at least 99%, or 100% identity to SEQ ID NO: 29; and/or
(b) the VL domain comprises a sequence having at least 70%, at least 75%, at least 80%, at least 85%, at least 90%, at least 95%, at least 97%, at least 99%, or 100% identity to SEQ ID NO: 30;
optionally wherein the antibody or fragment thereof comprises:
(i) a constant heavy chain comprising a sequence having at least 70%, at least 75%, at least 80%, at least 85%, at least 90%, at least 95%, at least 97%, at least 99%, or 100% identity to SEQ ID NO: 31; and/or
(ii) a constant light chain comprising a sequence having at least 70%, at least 75%, at least 80%, at least 85%, at least 90%, at least 95%, at least 97%, at least 99%, or 100% identity to SEQ ID NO: 32.

7. The modulator for use according to claim 4, wherein the anti-CFC1 antibody is:
(i) antibody clone MM0205-8D12;
or
(ii) the anti-CFC1 antibody M09 clone 2G4.

8. A polynucleotide for use in treating neuroendocrine neoplasms, wherein the polynucleotide comprises a nucleic acid sequence encoding the modulator as defined in any of claims 1 to 7.

9. The polynucleotide for use according to claim 8, wherein the nucleic acid sequence is an RNA sequence.

10. A vector for use in treating neuroendocrine neoplasms, wherein the vector comprises the polynucleotide as defined in claim 8 or claim 9.

11. A composition for use in treating neuroendocrine neoplasms, wherein the composition comprises the modulator of any of claims 1 to 7, or the polynucleotide as defined in claim 8 or claim 9, or the vector as defined in claim 10.

12. A pharmaceutical composition for use in treating neuroendocrine neoplasms, wherein the pharmaceutical composition comprises the modulator of any of claims 1 to 7, the polynucleotide as defined in claim 8 or claim 9, or the vector as defined in claim 10, or the composition for use as defined in claim 11; together with a pharmaceutically acceptable carrier, diluent or excipient.

13. The pharmaceutical composition for use according to claim 12, further comprising a vesicle, a nanoparticle, a lipid nanoparticle (LNP), a liposome or a polymeric mixture; optionally wherein the modulator, the polynucleotide, the vector, or the composition is encapsulated within the vesicle, nanoparticle, LNP, liposome or polymeric mixture.

14. The modulator for use according to any of claims 1 to 7, the polynucleotide for use according to claim 8 or claim 9, the vector for use according to claim 10, the composition for use according to claim 11, or the pharmaceutical composition for use according to claim 12 or claim 13; wherein the neuroendocrine neoplasm is a tumour or cancer expressing CFC1, wherein:
(i) the expression of CFC1 is increased compared to the expression of CFC1 by a non-cancerous tissue; and/or
(ii) wherein the neuroendocrine neoplasm is selected from the list consisting of: a colorectal neuroendocrine tumor, a pancreatic neuroendocrine tumor, a gastroenteropancreatic (GEP) tumour, and a pulmonal neuroendocrine tumor.

15. The modulator for use according to any of claims 1 to 7, the polynucleotide for use according to claim 8 or claim 9, the vector for use according to claim 10, the composition for use according to claim 11, or the pharmaceutical composition for use according to claim 12 or claim 13, or the modulator for use, the polynucleotide for use, the vector for use, the composition for use, or the pharmaceutical composition for use according to claim 14, wherein the neuroendocrine neoplasm is not neuroblastoma.
